# EUROPEAN PATENT APPLICATION

(11) **EP 4 582 540 A1**
(43) Date of publication of application: **09.07.2025**
(21) Application number: 23860446.6
(22) Date of filing: 30.08.2023
(51) Int. Cl.: C12N 15/10, C12N 15/11

(54) **METHOD FOR PRODUCING NUCLEIC ACID MOLECULE**

(30) Priority: 31.08.2022 JP 2022138632
(71) Applicant: Genedesign, Inc., Ibaraki-shi, Osaka 567-0085 (JP)
(72) Inventor: SANOSAKA, Masato, Ibaraki-shi, Osaka 567-0085 (JP); SAITO, Emi, Ibaraki-shi, Osaka 567-0085 (JP); NANKAI, Hirokazu, Ibaraki-shi, Osaka 567-0085 (JP)
(74) Representative: Weickmann & Weickmann PartmbB
(86) International application number: PCT/JP2023/031631
(87) International publication number: WO 2024/048684

(57) **Abstract**

The present disclosure provides a method for producing a nucleic acid molecule. Specifically, the present disclosure achieves process shortening and a dramatic improvement in ligation efficiency by using nucleic acid splints. That is, the present disclosure provides a method for producing a polynucleotide molecule having a specific sequence, the method including: 1) providing two or more polynucleotide fragment molecules including a portion of the specific sequence; and 2) ligating the two or more polynucleotide fragment molecules in the presence of two or more nucleic acid splints, wherein each sequence of the two or more polynucleotide fragment molecules when assembled constitutes the specific sequence, the nucleic acid splints are substantially complementary to the polynucleotide molecule, and at least two of the nucleic acid splints include a sequence which is substantially free of deletions when aligned with the specific sequence.

## Description

### Technical Field

The present disclosure relates to ligation reactions for the synthesis of long oligonucleotides or polynucleotides or variants thereof and applications of the ligation reactions.

### Background Art

DNA cleavage and ligation are one of the most important basic techniques in genetic engineering, and there are various techniques. RNA cleavage and ligation are also one of important basic techniques, and there are various techniques.

### Summary of Invention

### Solution to Problem

The present disclosure has achieved process shortening, dramatic efficiency in ligation using a nucleic acid splint. That is, the present disclosure has found that, in a nucleic acid ligation reaction, by adding two or more nucleic acid splints having a sequence complementary to the nucleic acid and substantially eliminating deletion, the ligation efficiency between the 3' end side of the nucleic acid and the 5' end side of the nucleic acid is dramatically improved.

The present invention provides, for example, the following items.

### (Item 1)

A method for producing a polynucleotide molecule having a specific sequence, the method including:
1) providing two or more polynucleotide fragment molecules including a portion of the specific sequence; and 2) ligating the two or more polynucleotide fragment molecules in the presence of two or more nucleic acid splints, in which

each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

### (Item 2A)

The method according to any one of the preceding items, in which there are three or more of the polynucleotide fragment molecules used.

### (Item 2B)

The method according to any one of the preceding items, in which there are four or more of the polynucleotide fragment molecules used.

### (Item 2C)

The method according to any one of the preceding items, in which there are five or more of the polynucleotide fragment molecules used.

### (Item 2D)

The method according to any one of the preceding items, in which there are 10 or more of the polynucleotide fragment molecules used.

### (Item 2E)

The method according to any one of the preceding items, in which there are 50 or less of the polynucleotide fragment molecules used.

### (Item 2AA)

The method according to any one of the preceding items, in which the polynucleotide fragment molecules are RNA or modified RNA, the nucleic acid splints are DNA, and the ligation is performed with an RNA ligase.

### (Item 2AB)

The method according to any one of the preceding items, in which the modification includes halo (fluoro, chloro, iodide, and the like), substituted (including alkoxy and the like) or unsubstituted alkyl (for example, methyl), nucleic acid or modified nucleic acid (for example, BNA, LNA), phosphorothioate and the like.

### (Item 2AC)

The method according to any one of the preceding items, in which the polynucleotide fragment molecule is monophosphorylated.

### (Item 2AD)

The method according to any one of the preceding items, in which the polynucleotide fragment molecule is a monophosphorylated RNA.

### (Item 2AE)

The method according to any one of the preceding items, in which the polynucleotide fragment molecule is provided in at least one method selected from a group consisting of chemical synthesis and transcriptional synthesis.

### (Item 2AF)

The method according to any one of the preceding items, in which the polynucleotide fragment molecule is provided in both a chemical synthesis method and a transcriptional synthesis method.

### (Item 2AG)

The method according to any one of the preceding items, including providing the polynucleotide fragment molecule by transcriptional synthesis, and monophosphorylating a 5' end of the polynucleotide fragment molecule after the transcriptional synthesis, in which the ligation is performed using an RNA ligase.

### (Item 3)

The method according to any one of the preceding items, in which the nucleic acid splints are 8 mer or more complementary to the polynucleotide molecule.

### (Item 4)

The method according to any one of the preceding items, in which a distance between a nucleic acid splint aligned with the specific sequence and a nucleic acid splint adjacent to the nucleic acid splint aligned with the specific sequence is 2 bases or less.

### (Item 5)

The method according to any one of the preceding items, in which the deletions are 2 bases or less.

### (Item 6A)

The method according to any one of the preceding items, in which the nucleic acid splints are 10 mer to 50 mer.

### (Item 6B)

The method according to any one of the preceding items, in which the polynucleotide fragment molecules are 10 mer to 50 mer.

### (Item 7)

The method according to any one of the preceding items, in which the nucleic acid splint has a sequence that protrudes from a joining point of a polynucleotide fragment molecule.

### (Item 7A)

The method according to any one of the preceding items, in which the nucleic acid splint has a sequence that protrudes 2 to 8 mer or more from a joining point of a polynucleotide fragment molecule.

### (Item 7B)

The method according to any one of the preceding items, in which the nucleic acid splint has a sequence that protrudes 5 mer or more from a joining point of a polynucleotide fragment molecule.

### (Item 7C)

The method according to any one of the preceding items, in which the two or more nucleic acid splints and the polynucleotide fragment molecules have a sequence designed to be shifted by one or more bases when aligned with the specific sequence.

### (Item 8)

The method according to any one of the preceding items, in which at least one end of the polynucleotide fragment molecule has an association with a carrier.

### (Item 9)

The method according to any one of the preceding items, in which the carrier is polystyrene, CPG (Controlled pore glass), or magnetic beads.

### (Item 9A)

The method according to any one of the preceding items, in which the ligation is performed through enzymatic ligation reactions.

### (Item 9B)

The method according to any one of the preceding items, in which the enzymatic ligation reactions are performed simultaneously or at different times.

### (Item 9C)

The method according to any one of the preceding items, in which the enzymatic ligation reactions are performed simultaneously.

### (Item 9D)

The method according to any one of the preceding items, in which in the enzymatic ligation reactions, (A) one ligation reaction is performed in a combination that results in a total extension which is shorter than the specific sequence of the polynucleotide fragment molecule, then the next ligation reaction is performed using a product of the one ligation reaction, and (B) (A) is repeated as necessary.

### (Item 9E)

The method according to any one of the preceding items, in which the total extension is a combination of 175 to 200 mer.

### (Item 10)

A polynucleotide molecule produced by the method according to any one of the preceding items.

### <Kit>

### (Item A1)

A kit for producing a polynucleotide molecule having a specific sequence, the kit including:
1) two or more polynucleotide fragment molecules including a portion of the specific sequence; and
2) two or more nucleic acid splints, in which

each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

### (Item A1A)

The kit according to any one of the preceding items, in which there are three or more of the polynucleotide fragment molecules used.

### (Item A1B)

The kit according to any one of the preceding items, in which there are four or more of the polynucleotide fragment molecules used.

### (Item A1C)

The kit according to any one of the preceding items, in which there are five or more of the polynucleotide fragment molecules used.

### (Item A1D)

The kit according to any one of the preceding items, in which there are 10 or more of the polynucleotide fragment molecules used.

### (Item A1E)

The kit according to any one of the preceding items, in which there are 50 or less of the polynucleotide fragment molecules used.

### (Item A1AA)

The kit according to any one of the preceding items, in which the polynucleotide fragment molecules are RNA or modified RNA, the nucleic acid splints are DNA, and the ligation is performed with an RNA ligase.

### (Item A1AB)

The kit according to any one of the preceding items, in which the modification includes halo (fluoro, chloro, iodide, and the like), substituted (including alkoxy and the like) or unsubstituted alkyl (for example, methyl), nucleic acid or modified nucleic acid (for example, BNA, LNA), phosphorothioate and the like.

### (Item A1AC)

The kit according to any one of the preceding items, in which the polynucleotide fragment molecule is a monophosphorylated.

### (Item A1AD)

The kit according to any one of the preceding items, in which the polynucleotide fragment molecule is a monophosphorylated RNA.

### (Item A1AE)

The kit according to any one of the preceding items, in which the polynucleotide fragment molecule is provided in at least one kit selected from a group consisting of kits of chemical synthesis and transcriptional synthesis.

### (Item A1AF)

The kit according to any one of the preceding items, in which the polynucleotide fragment molecule is provided in both a kit of chemical synthesis and a kit of transcriptional synthesis.

### (Item A1AG)

The kit according to any one of the preceding items, including providing the polynucleotide fragment molecule by transcriptional synthesis, and monophosphorylating a 5' end of the polynucleotide fragment molecule after the transcriptional synthesis, in which the ligation is performed using an RNA ligase.

### (Item A2)

The kit according to any one of the preceding items, in which the nucleic acid splints are 8 mer or more complementary to the polynucleotide molecule.

### (Item A3)

The kit according to any one of the preceding items, in which a distance between a nucleic acid splint aligned with the specific sequence and an adjacent nucleic acid splint aligned with the specific sequence is 2 bases or less.

### (Item A4)

The kit according to any one of the preceding items, in which the deletions are 2 bases or less.

### (Item A5)

The kit according to any one of the preceding items, in which the nucleic acid splints are 10 mer to 50 mer.

### (Item A6)

The kit according to any one of the preceding items, in which the polynucleotide fragment molecules are 10 mer to 50 mer.

### (Item A7)

The kit according to any one of the preceding items, in which the nucleic acid splint has a sequence that protrudes from a joining point of a polynucleotide fragment molecule.

### (Item A7A)

The kit according to any one of the preceding items, in which the nucleic acid splint has a sequence that protrudes 2 to 8 mer or more from a joining point of a polynucleotide fragment molecule.

### (Item A7B)

The kit according to any one of the preceding items, in which the nucleic acid splint has a sequence that protrudes 5 mer or more from a j oining point of a polynucleotide fragment molecule.

### (Item A7C)

The kit according to any one of the preceding items, in which the two or more nucleic acid splints and the polynucleotide fragment molecules have a sequence designed to be shifted by one or more bases when aligned with the specific sequence.

### (Item A8)

The kit according to any one of the preceding items, in which at least one end of the polynucleotide fragment molecule has an association with a carrier.

### (Item A9)

The kit according to any one of the preceding items, in which the carrier is polystyrene, CPG (Controlled pore glass), or magnetic beads.

### (Item A9A)

The kit according to any one of the preceding items, in which the ligation is performed through enzymatic ligation reactions.

### (Item A9B)

The kit according to any one of the preceding items, in which the enzymatic ligation reactions are performed simultaneously or at different times.

### (Item A9C)

The kit according to any one of the preceding items, in which the enzymatic ligation reactions are performed simultaneously.

### (Item A9D)

The kit according to any one of the preceding items, in which in the enzymatic ligation reactions, (A) one ligation reaction is performed in a combination that results in a total extension which is shorter than the specific sequence of the polynucleotide fragment molecule, then the next ligation reaction is performed using a product of the one ligation reaction, and (B) (A) is repeated as necessary.

### (Item A9E)

The kit according to any one of the preceding items, in which the total extension is a combination of 175 to 200 mer.

### <Material provision>

### (Item B1)

A method for producing a polynucleotide molecule having a specific sequence, the method including:
A) designing two or more polynucleotide fragment molecules including a portion of the desired sequence based on the specific sequence;
B) designing two or more nucleic acid splints based on the specific sequence;
C) providing the polynucleotide fragment molecules and the nucleic acid splints which have been designed; and
D) ligating the polynucleotide fragment molecules which have been designed in the presence of the nucleic acid splints which have been designed, in which

each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

### (Item B2)

A method for producing a polypeptide fragment and a nucleic acid splint for producing a polynucleotide molecule having a specific sequence, the method including:
A) designing two or more polynucleotide fragment molecules including a portion of the desired sequence based on the specific sequence;
B) designing two or more nucleic acid splints based on the specific sequence; and
C) producing the polynucleotide fragment molecules and the two or more nucleic acid splints which have been designed, in which

each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

### (Item B2A)

The method according to any one of the preceding items, in which there are three or more of the polynucleotide fragment molecules used.

### (Item B2B)

The method according to any one of the preceding items, in which there are four or more of the polynucleotide fragment molecules used.

### (Item B2C)

The method according to any one of the preceding items, in which there are five or more of the polynucleotide fragment molecules used.

### (Item B2D)

The method according to any one of the preceding items, in which there are 10 or more of the polynucleotide fragment molecules used.

### (Item B2E)

The method according to any one of the preceding items, in which there are 50 or less of the polynucleotide fragment molecules used.

### (Item B2AA)

The method according to any one of the preceding items, in which the polynucleotide fragment molecules are RNA or modified RNA, the nucleic acid splints are DNA, and the ligation is performed with an RNA ligase.

### (Item B2AB)

The method according to any one of the preceding items, in which the modification includes halo (fluoro, chloro, iodide, and the like), substituted (including alkoxy and the like) or unsubstituted alkyl (for example, methyl), nucleic acid or modified nucleic acid (for example, BNA, LNA), phosphorothioate and the like.

### (Item B2AC)

The method according to any one of the preceding items, in which the polynucleotide fragment molecule is monophosphorylated.

### (Item B2AD)

The method according to any one of the preceding items, in which the polynucleotide fragment molecule is a monophosphorylated of RNA.

### (Item B2AE)

The method according to any one of the preceding items, in which the polynucleotide fragment molecule is provided in at least one method selected from a group consisting of chemical synthesis and transcriptional synthesis.

### (Item B2AF)

The method according to any one of the preceding items, in which the polynucleotide fragment molecule is provided in both a chemical synthesis method and a transcriptional synthesis method.

### (Item B2AG)

The method according to any one of the preceding items, including providing the polynucleotide fragment molecule by transcriptional synthesis, and monophosphorylating a 5' end of the polynucleotide fragment molecule after the transcriptional synthesis, in which the ligation is performed using an RNA ligase.

### (Item B3)

The method according to any one of the preceding items, in which the nucleic acid splints are 8 mer or more complementary to the polynucleotide molecule.

### (Item B4)

The method according to any one of the preceding items, in which a distance between a nucleic acid splint aligned with the specific sequence and a nucleic acid splint adjacent to the nucleic acid splint aligned with the specific sequence is 2 bases or less.

### (Item B5)

The method according to any one of the preceding items, in which the deletions are 2 bases or less.

### (Item B6)

The method according to any one of the preceding items, in which the nucleic acid splints are 10 mer to 50 mer.

### (Item B7)

The method according to any one of the preceding items, in which the polynucleotide fragment molecules are 10 mer to 50 mer.

### (Item B7-1)

The method according to any one of the preceding items, in which the nucleic acid splint has a sequence that protrudes from a joining point of a polynucleotide fragment molecule.

### (Item B7A)

The method according to any one of the preceding items, in which the nucleic acid splint has a sequence that protrudes 2 to 8 mer or more from a joining point of a polynucleotide fragment molecule.

### (Item B7B)

The method according to any one of the preceding items, in which the nucleic acid splint has a sequence that protrudes 5 mer or more from a joining point of a polynucleotide fragment molecule.

### (Item B7C)

The method according to any one of the preceding items, in which the two or more nucleic acid splints and the polynucleotide fragment molecules have a sequence designed to be shifted by one or more bases when aligned with the specific sequence.

### (Item B8)

The method according to any one of the preceding items, in which at least one end of the polynucleotide fragment molecule has an association with a carrier.

### (Item B9)

The method according to any one of the preceding items, in which the carrier is polystyrene, CPG (Controlled pore glass), or magnetic beads.

### (Item B9A)

The method according to any one of the preceding items, in which the ligation is performed through enzymatic ligation reactions.

### (Item B9B)

The method according to any one of the preceding items, in which the enzymatic ligation reactions are performed simultaneously or at different times.

### (Item B9C)

The method according to any one of the preceding items, in which the enzymatic ligation reactions are performed simultaneously.

### (Item B9D)

The method according to any one of the preceding items, in which in the enzymatic ligation reactions, (A) one ligation reaction is performed in a combination that results in a total extension which is shorter than the specific sequence of the polynucleotide fragment molecule, then the next ligation reaction is performed using a product of the one ligation reaction, and (B) (A) is repeated as necessary.

### (Item B9E)

The method according to any one of the preceding items, in which the total extension is a combination of 175 to 200 mer.

### <Design system>

### (Item C1)

A method of designing a polypeptide fragment and a nucleic acid splint for producing a polynucleotide molecule having a specific sequence, the method including:
A) designing two or more polynucleotide fragment molecules including a portion of the desired sequence based on the specific sequence; and
B) designing two or more nucleic acid splints based on the specific sequence, in which

each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

### (Item C2A)

The method according to any one of the preceding items, in which there are three or more of the polynucleotide fragment molecules used.

### (Item C2B)

The method according to any one of the preceding items, in which there are four or more of the polynucleotide fragment molecules used.

### (Item C2C)

The method according to any one of the preceding items, in which there are five or more of the polynucleotide fragment molecules used.

### (Item C2D)

The method according to any one of the preceding items, in which there are 10 or more of the polynucleotide fragment molecules used.

### (Item C2E)

The method according to any one of the preceding items, in which there are 50 or less of the polynucleotide fragment molecules used.

### (Item C2AA)

The method according to any one of the preceding items, in which the polynucleotide fragment molecules are RNA or modified RNA, the nucleic acid splints are DNA, and the ligation is performed with an RNA ligase.

### (Item C2AB)

The method according to any one of the preceding items, in which the modification includes halo (fluoro, chloro, iodide, and the like), substituted (including alkoxy and the like) or unsubstituted alkyl (for example, methyl), nucleic acid or modified nucleic acid (for example, BNA, LNA), phosphorothioate and the like.

### (Item C2AC)

The method according to any one of the preceding items, in which the polynucleotide fragment molecule is monophosphorylated.

### (Item C2AD)

The method according to any one of the preceding items, in which the polynucleotide fragment molecule is monophosphorylatedRNA.

### (Item C2AE)

The method according to any one of the preceding items, in which the polynucleotide fragment molecule is provided in at least one method selected from a group consisting of chemical synthesis and transcriptional synthesis.

### (Item C2AF)

The method according to any one of the preceding items, in which the polynucleotide fragment molecule is provided in both a chemical synthesis method and a transcriptional synthesis method.

### (Item C2AG)

The method according to any one of the preceding items, including providing the polynucleotide fragment molecule by transcriptional synthesis, and monophosphorylating a 5' end of the polynucleotide fragment molecule after the transcriptional synthesis, in which the ligation is performed using an RNA ligase.

### (Item C2)

The method according to any one of the preceding items, in which the nucleic acid splints are 8 mer or more complementary to the polynucleotide molecule.

### (Item C3)

The method according to any one of the preceding items, in which a distance between a nucleic acid splint aligned with the specific sequence and a nucleic acid splint adjacent to the nucleic acid splint aligned with the specific sequence is 2 bases or less.

### (Item C4)

The method according to any one of the preceding items, in which the deletions are 2 bases or less.

### (Item C5)

The method according to any one of the preceding items, in which the nucleic acid splints are 10 mer to 50 mer.

### (Item C6)

The method according to any one of the preceding items, in which the polynucleotide fragment molecules are 10 mer to 50 mer.

### (Item C7)

The method according to any one of the preceding items, in which the nucleic acid splint has a sequence that protrudes from a joining point of a polynucleotide fragment molecule.

### (Item C7A)

The method according to any one of the preceding items, in which the nucleic acid splint has a sequence that protrudes 2 to 8 mer or more from a joining point of a polynucleotide fragment molecule.

### (Item C7B)

The method according to any one of the preceding items, in which the nucleic acid splint has a sequence that protrudes 5 mer or more from a joining point of a polynucleotide fragment molecule.

### (Item C7C)

The method according to any one of the preceding items, in which the two or more nucleic acid splints and the polynucleotide fragment molecules have a sequence designed to be shifted by one or more bases when aligned with the specific sequence.

### (Item C8)

The method according to any one of the preceding items, in which at least one end of the polynucleotide fragment molecule has an association with a carrier.

### (Item C9)

The method according to any one of the preceding items, in which the carrier is polystyrene, CPG (Controlled pore glass), or magnetic beads.

### (Item C9A)

The method according to any one of the preceding items, in which the ligation is performed through enzymatic ligation reactions.

### (Item C9B)

The method according to any one of the preceding items, in which the enzymatic ligation reactions are performed simultaneously or at different times.

### (Item C9C)

The method according to any one of the preceding items, in which the enzymatic ligation reactions are performed simultaneously.

### (Item C9D)

The method according to any one of the preceding items, in which in the enzymatic ligation reactions, (A) one ligation reaction is performed in a combination that results in a total extension which is shorter than the specific sequence of the polynucleotide fragment molecule, then the next ligation reaction is performed using a product of the one ligation reaction, and (B) (A) is repeated as necessary.

### (Item C9E)

The method according to any one of the preceding items, in which the total extension is a combination of 175 to 200 mer.

### <Program>

### (Item D1)

A program for causing a computer to execute a method of designing a polypeptide fragment and a nucleic acid splint for producing a polynucleotide molecule having a specific sequence, the method including:
A) designing two or more polynucleotide fragment molecules including a portion of the desired sequence based on the specific sequence; and
B) designing two or more nucleic acid splints based on the specific sequence, in which

each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

### (Item D2A)

The program according to any one of the preceding items, in which there are three or more of the polynucleotide fragment molecules used.

### (Item D2B)

The program according to any one of the preceding items, in which there are four or more of the polynucleotide fragment molecules used.

### (Item D2C)

The program according to any one of the preceding items, in which there are five or more of the polynucleotide fragment molecules used.

### (Item D2D)

The program according to any one of the preceding items, in which there are 10 or more of the polynucleotide fragment molecules used.

### (Item D2E)

The program according to any one of the preceding items, in which there are 50 or less of the polynucleotide fragment molecules used.

### (Item D2AA)

The program according to any one of the preceding items, in which the polynucleotide fragment molecules are RNA or modified RNA, the nucleic acid splints are DNA, and the ligation is performed with an RNA ligase.

### (Item D2AB)

The program according to any one of the preceding items, in which the modification includes halo (fluoro, chloro, iodide, and the like), substituted (including alkoxy and the like) or unsubstituted alkyl (for example, methyl), nucleic acid or modified nucleic acid (for example, BNA, LNA), phosphorothioate and the like.

### (Item D2AC)

The program according to any one of the preceding items, in which the polynucleotide fragment molecule is monophosphorylated.

### (Item D2AD)

The program according to any one of the preceding items, in which the polynucleotide fragment molecule is a monophosphorylated RNA.

### (Item D2AE)

The program according to any one of the preceding items, in which the polynucleotide fragment molecule is provided in at least one method selected from a group consisting of chemical synthesis and transcriptional synthesis.

### (Item D2AF)

The program according to any one of the preceding items, in which the polynucleotide fragment molecule is provided in both a chemical synthesis method and a transcriptional synthesis method.

### (Item D2AG)

The program according to any one of the preceding items, including providing the polynucleotide fragment molecule by transcriptional synthesis, and monophosphorylating a 5' end of the polynucleotide fragment molecule after the transcriptional synthesis, in which the ligation is performed using an RNA ligase.

### (Item D2)

The program according to any one of the preceding items, in which the nucleic acid splints are 8 mer or more complementary to the polynucleotide molecule.

### (Item D3)

The program according to any one of the preceding items, in which a distance between a nucleic acid splint aligned with the specific sequence and a nucleic acid splint adjacent to the nucleic acid splint aligned with the specific sequence is 2 bases or less.

### (Item D4)

The program according to any one of the preceding items, in which the deletions are 2 bases or less.

### (Item D5)

The program according to any one of the preceding items, in which the nucleic acid splints are 10 mer to 50 mer.

### (Item D6)

The program according to item D1, in which the polynucleotide fragment molecules are 10 mer to 50 mer.

### (Item D7)

The program according to any one of the preceding items, in which the nucleic acid splint has a sequence that protrudes from a joining point of a polynucleotide fragment molecule.

### (Item D7A)

The program according to any one of the preceding items, in which the nucleic acid splint has a sequence that protrudes 2 to 8 mer or more from a joining point of a polynucleotide fragment molecule.

### (Item D7B)

The program according to any one of the preceding items, in which the nucleic acid splint has a sequence that protrudes 5 mer or more from a joining point of a polynucleotide fragment molecule.

### (Item D7C)

The program according to any one of the preceding items, in which the two or more nucleic acid splints and the polynucleotide fragment molecules have a sequence designed to be shifted by one or more bases when aligned with the specific sequence.

### (Item D8)

The program according to any one of the preceding items, in which at least one end of the polynucleotide fragment molecule has an association with a carrier.

### (Item D9)

The program according to any one of the preceding items, in which the carrier is polystyrene, CPG (Controlled pore glass), or magnetic beads.

### (Item D9A)

The program according to any one of the preceding items, in which the ligation is performed through enzymatic ligation reactions.

### (Item D9B)

The program according to any one of the preceding items, in which the enzymatic ligation reactions are performed simultaneously or at different times.

### (Item D9C)

The program according to any one of the preceding items, in which the enzymatic ligation reactions are performed simultaneously.

### (Item D9D)

The program according to any one of the preceding items, in which in the enzymatic ligation reactions, (A) one ligation reaction is performed in a combination that results in a total extension which is shorter than the specific sequence of the polynucleotide fragment molecule, then the next ligation reaction is performed using a product of the one ligation reaction, and (B) (A) is repeated as necessary.

### (Item D9E)

The program according to any one of the preceding items, in which the total extension is a combination of 175 to 200 mer.

### <Recording medium>

### (Item E1)

A recording medium storing a program for causing a computer to execute a method of designing a polypeptide fragment and a nucleic acid splint for producing a polynucleotide molecule having a specific sequence, the method including:
A) designing two or more polynucleotide fragment molecules including a portion of the desired sequence based on the specific sequence; and
B) designing two or more nucleic acid splints based on the specific sequence, in which

each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

### (Item E2A)

The recording medium according to any one of the preceding items, in which there are three or more of the polynucleotide fragment molecules used.

### (Item E2B)

The recording medium according to any one of the preceding items, in which there are four or more of the polynucleotide fragment molecules used.

### (Item E2C)

The recording medium according to any one of the preceding items, in which there are five or more of the polynucleotide fragment molecules used.

### (Item E2D)

The recording medium according to any one of the preceding items, in which there are 10 or more of the polynucleotide fragment molecules used.

### (Item E2E)

The recording medium according to any one of the preceding items, in which there are 50 or less of the polynucleotide fragment molecules used.

### (Item E2AA)

The recording medium according to any one of the preceding items, in which the polynucleotide fragment molecules are RNA or modified RNA, the nucleic acid splints are DNA, and the ligation is performed with an RNA ligase.

### (Item E2AB)

The recording medium according to any one of the preceding items, in which the modification includes halo (fluoro, chloro, iodide, and the like), substituted (including alkoxy and the like) or unsubstituted alkyl (for example, methyl), nucleic acid or modified nucleic acid (for example, BNA, LNA), phosphorothioate and the like.

### (Item E2AC)

The recording medium according to any one of the preceding items, in which the polynucleotide fragment molecule is monophosphorylated.

### (Item E2AD)

The recording medium according to any one of the preceding items, in which the polynucleotide fragment molecule is a monophosphorylated RNA.

### (Item E2AE)

The recording medium according to any one of the preceding items, in which the polynucleotide fragment molecule is provided in at least one method selected from a group consisting of chemical synthesis and transcriptional synthesis.

### (Item E2AF)

The recording medium according to any one of the preceding items, in which the polynucleotide fragment molecule is provided in both a chemical synthesis method and a transcriptional synthesis method.

### (Item E2AG)

The recording medium according to any one of the preceding items, including providing the polynucleotide fragment molecule by transcriptional synthesis, and monophosphorylating a 5' end of the polynucleotide fragment molecule after the transcriptional synthesis, in which the ligation is performed using an RNA ligase.

### (Item E2)

The recording medium according to any one of the preceding items, in which the nucleic acid splints are 8 mer or more complementary to the polynucleotide molecule.

### (Item E3)

The recording medium according to any one of the preceding items, in which a distance between a nucleic acid splint aligned with the specific sequence and a nucleic acid splint adjacent to the nucleic acid splint aligned with the specific sequence is 2 bases or less.

### (Item E4)

The recording medium according to any one of the preceding items, in which the deletions are 2 bases or less.

### (Item E5)

The recording medium according to any one of the preceding items, in which the nucleic acid splints are 10 mer to 50 mer.

### (Item E6)

The recording medium according to any one of the preceding items, in which the polynucleotide fragment molecules are 10 mer to 50 mer.

### (Item E7)

The recording medium according to any one of the preceding items, in which the nucleic acid splint has a sequence that protrudes from a joining point of a polynucleotide fragment molecule.

### (Item E7A)

The recording medium according to any one of the preceding items, in which the nucleic acid splint has a sequence that protrudes 2 to 8 mer or more from a joining point of a polynucleotide fragment molecule.

### (Item E7B)

The recording medium according to any one of the preceding items, in which the nucleic acid splint has a sequence that protrudes 5 mer or more from a joining point of a polynucleotide fragment molecule.

### (Item E7C)

The recording medium according to any one of the preceding items, in which the two or more nucleic acid splints and the polynucleotide fragment molecules have a sequence designed to be shifted by one or more bases when aligned with the specific sequence.

### (Item E8)

The recording medium according to any one of the preceding items, in which at least one end of the polynucleotide fragment molecule has an association with a carrier.

### (Item E9)

The recording medium according to any one of the preceding items, in which the carrier is polystyrene, CPG (Controlled pore glass), or magnetic beads.

### (Item E9A)

The recording medium according to any one of the preceding items, in which the ligation is performed through enzymatic ligation reactions.

### (Item E9B)

The recording medium according to any one of the preceding items, in which the enzymatic ligation reactions are performed simultaneously or at different times.

### (Item E9C)

The recording medium according to any one of the preceding items, in which the enzymatic ligation reactions are performed simultaneously.

### (Item E9D)

The recording medium according to any one of the preceding items, in which in the enzymatic ligation reactions, (A) one ligation reaction is performed in a combination that results in a total extension which is shorter than the specific sequence of the polynucleotide fragment molecule, then the next ligation reaction is performed using a product of the one ligation reaction, and (B) (A) is repeated as necessary.

### (Item E9E)

The recording medium according to any one of the preceding items, in which the total extension is a combination of 175 to 200 mer.

In the present disclosure, it is intended that one or a plurality of the above features may be provided in further combination in addition to the specified combinations. Still further embodiments and advantages of the present disclosure will be appreciated by those skilled in the art upon reading and understanding the following detailed description as necessary.

### Advantageous Effects of Invention

Nucleic acid splints are used to provide a ligation reaction of nucleic acids using complementary sequences which are substantially free of deletions. By using the present ligation reaction, the yield can be improved as compared with the conventional ligation reactions. The present disclosure also provides a technique capable of ligating DNA to mRNA and its 3' end, and provides a technique with improved efficiency. The present disclosure also provides a technique in which an in vitro virus method can also be utilized, and provides a more efficient processing technique for specific cleavage and binding of DNA, which contributes to the development of genetic engineering.

### Brief Description of Drawings

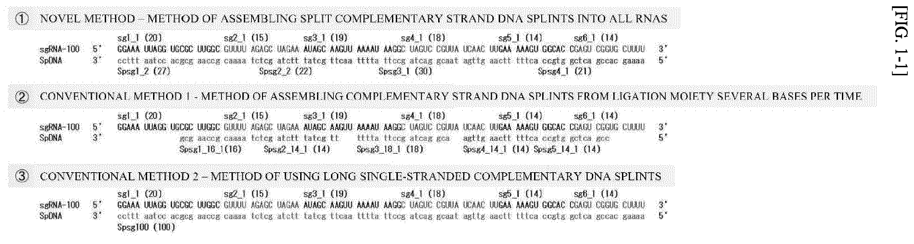

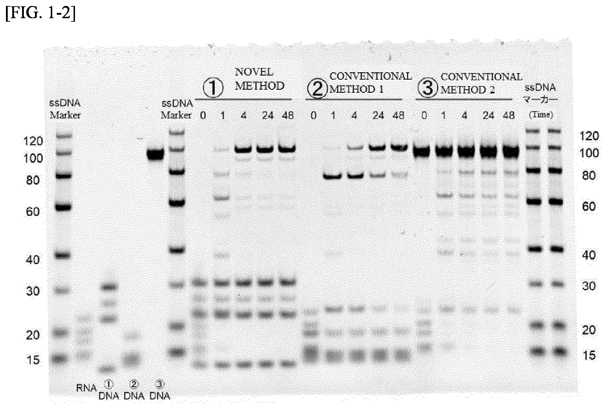

[FIG. 1-1] FIG. 1-1 shows a sequence used in Example 1.
[FIG. 1-2] FIG. 1-2 shows results of Denatured-PAGE of enzymatic ligation with ssRNA (short RNA) 100 mer 6-split RNA ligase 2.
[FIG. 2] FIG. 2 shows results of measurement by HPLC/LC-MS of enzymatic ligation with ssRNA (short RNA) 100 mer 6-split RNA ligase 2.
[FIG. 3] FIG. 3 shows results of reaction tracking by UHPLC of enzymatic ligation with ssRNA (short RNA) 100 mer 6-split RNA ligase 2.
[FIG. 4] FIG. 4 shows results of Denatured-PAGE of enzymatic ligation with ssRNA 220 mer 10-split RNA ligase 2.
[FIG. 5] FIG. 5 shows results of UHPLC/LC-MS of Example 4.
[FIG. 6-1] FIG. 6-1 shows a sequence used in Example 5.
[FIG. 6-2] FIG. 6-2 shows results of Denatured-PAGE of Example 5.
[FIG. 7-1] FIG. 7-1 shows a sequence used in Example 1.
[FIG. 7-2] FIG. 7-2 shows results of Denatured-PAGE and results of activity measurement of Example 6.
[FIG. 8-1] FIG. 8-1 shows a sequence used in Example 6.
[FIG. 8-2] FIG. 8-2 shows a sequence used in Example 6.
[FIG. 8-3] FIG. 8-3 shows results of Denatured-PAGE and results of activity measurement of Example 6.
[FIG. 9] FIG. 9 shows a schematic diagram of the method of the present disclosure.

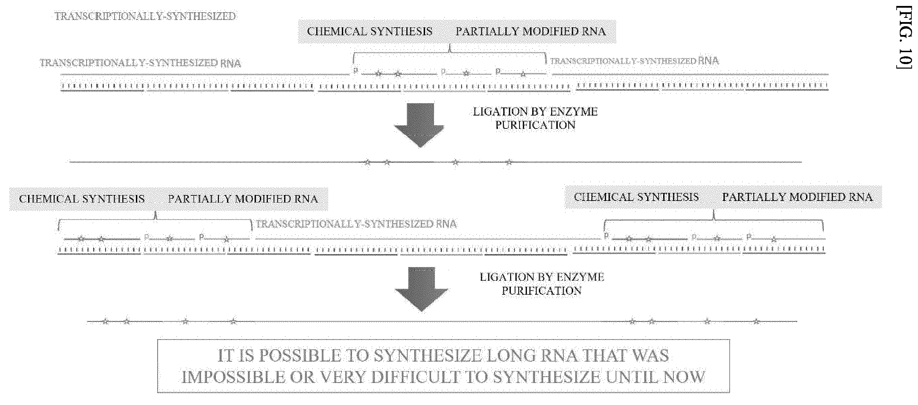

[FIG. 10] FIG. 10 shows a ligation method of transcriptionally synthesized long RNA and chemically synthesized RNA using the development method of the present disclosure. In the drawing, the stars in chemical synthesis indicate modification. In the method of the present disclosure, a nucleic acid splint (here, a DNA splint) is designed so as to form a complementary strand with the entire target sequence that is a specific sequence, and then the nucleic acid splint is divided instead of being a single strand unlike existing techniques, so that the strand length of the nucleic acid splint is extremely short as compared with single-stranded (ss) RNA, and purification becomes easy.
[FIG. 11] FIG. 11 shows an example of making strands to be templates for each other without using template DNA.
[FIG. 12] FIG. 12 shows an application example of an enzymatic ligation reaction by ssRNA 100 mer 6-split RNA ligase 2. For example, it can be used for cleaving and editing genomic DNA by the CRISPR-Cas9 method.
[FIG. 13] FIG. 13 shows an outline of a ligation test of transcripts and chemically synthesized RNA using a DNA splint.
[FIG. 14] FIG. 14 is a schematic diagram of a target substance and a starting substance.
[FIG. 15] FIG. 15 shows an outline of a ligation reaction test of transcripts and chemically synthesized RNA using a DNA splint.

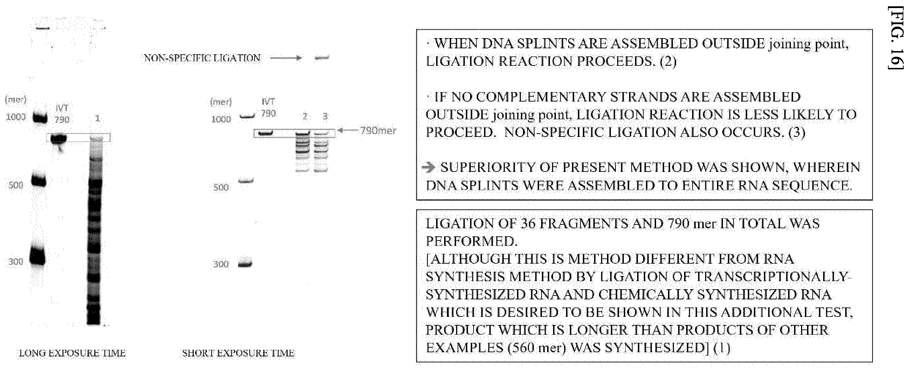

[FIG. 16] FIG. 16 shows results of Example 8. Ligation of 36 fragments and 790 mer in total was achieved. A product longer than that of the above Example (560 mer) could be synthesized using a technique other than the RNA synthesis method by ligation of transcriptionally synthesized RNA and chemically synthesized RNA (1). In addition, it was found that the ligation reaction proceeds when the DNA splint is assembled outside a joining point (1). In addition, the ligation reaction is less likely to proceed unless a complementary strand is assembled outside the joining point. Non-specific ligations were also found to occur (2).
[FIG. 17] FIG. 17 is a schematic diagram of an experiment of ligation of transcripts and chemically synthesized RNA using a DNA splint.
[FIG. 18] FIG. 18 shows results of an experiment of ligation of transcripts and chemically synthesized RNA using a DNA splint.

### Description of Embodiments

Hereinafter, the present disclosure will be described in more detail.

It is to be understood that throughout the entire description, the singular forms of expression also include the concept of the plural forms thereof, unless otherwise stated. Thus, it is to be understood that the singular articles (For example, "a", "an", "the", and the like in English) also include the plural concept thereof unless otherwise stated. It is also to be understood that the terms used in the present description are used in the sense commonly used in the art unless otherwise stated. Thus, unless defined otherwise, all technical and scientific terms used in the present description have the same meaning as commonly understood by those skilled in the art to which the present disclosure pertains. In the case of any contradiction, the present description (including the definitions) shall prevail.

### (Definitions)

As used herein, "nucleotide" and "nucleotide molecule" refer to a nucleoside in which a sugar moiety is a phosphate ester. As used herein, "nucleoside" refers to a compound in which a base and a sugar form an N-glycosidic bond. Those in which the sugar moiety is D-ribose are referred to as ribonucleotides, and are obtained by hydrolysis of RNA. Those in which the sugar moiety is D-2'-deoxyribose are referred to as deoxyribonucleotides, and are obtained by enzymatic degradation of DNA. As used herein, a nucleotide may be natural or non-natural, or may be artificially synthesized. A "nucleotide" may be modified, and modifications may be made in any of a base, a sugar moiety, and a phosphate group moiety, or a combination thereof, preferably the sugar moiety, the phosphate group moiety of the nucleotide, and/or the 3'position of the nucleotide may be modified with a phosphate group. In nucleotide variants, the 3' position of the nucleotide may be modified with a phosphate group, and at least one oxygen atom of the phosphate group may be substituted with a sulfur atom, boron (boranophosphated), a methyl group (methylated), or the like.

"Oligonucleotide," "oligonucleotide molecule," "polynucleotide," and "polynucleotide molecule" in the present description are used interchangeably and refer to two or more polymers of nucleotides, including polymers or oligomers of commonly used nucleotides. "Oligonucleotide", "oligonucleotide molecule", "polynucleotide" and "polynucleotide molecule" may also be referred to as "oligo", "nucleic acid" or "nucleic acid strand". "Oligonucleotide," "oligonucleotide molecule," "polynucleotide," and "polynucleotide molecule" may be modified.

As used herein, "modification" (also referred to as "modify", and in English, any of them usually corresponds to modification/modify, and includes the concepts of substitution, substitute, displacement, displace, replacement, and replace), as used in the context of a nucleic acid, refers to a state in which a constituent unit of the nucleic acid or a part or all of its end is replaced with another atomic group or a functional group is added. Those containing such "modifications" are referred to as "variants" (of DNA, RNA, nucleic acid, oligonucleotide, or the like). Modifications may be made to any of a base, a sugar moiety, and a phosphate group moiety, or a combination thereof at the nucleotide, preferably the sugar moiety, phosphate group moiety of the oligonucleotide or polynucleotide may be modified, and/or the 3' position of the 3' end of the oligonucleotide or polynucleotide may be modified with a phosphate group. In a variant of an oligonucleotide or polynucleotide, the 3' position of the 3' end of the oligonucleotide or polynucleotide may be modified with a phosphate group, and at least one oxygen atom of the phosphate group may be substituted with a sulfur atom. "Oligonucleotide variants" and "polynucleotide variants" in the present description are used interchangeably and refer to polymers or oligomers of nucleotides and/or nucleotide variants. Variants of oligonucleotides include all those capable of alkylation such as methylation, and include those including any modification disclosed in the present description (modification with various substituents). Other examples in the art include BNA (Bridged Nucleic Acid) and LNA (Locked Nucleic Acid). For variants such as artificial nucleic acids, see Wengel et al.,Eur.J.Org.Chem.2297-2321(2005). This document is incorporated in the present description as a reference. "Oligonucleotide", "oligonucleotide molecule", "polynucleotide" and "polynucleotide molecule" of the present disclosure may include such modifications.

As used herein, the "fragment molecule" refers to a molecule that is shorter than the entire target molecule when considering the molecule intended for synthesis in the synthesis technique of the present disclosure as a whole, and refers to a molecule that, upon ligation, generates the entire target molecule. In the case of a polynucleotide, a (whole) polynucleotide having a desired sequence can be produced by ligating fragment molecules of the polynucleotide.

As used herein, "deoxyribonucleic acid (DNA)" means a molecule including at least one deoxyribonucleotide monomer moiety. The DNA may be modified with any functional group, and examples of variants of the DNA include, but are not limited to, a cross-ligated nucleic acid: 2', 4'-BNA/LNA, 2', 4'-BNANC [N-Me], 2'-O, 4'-C-ethylene-cross-ligated nucleic acid (ENA), an amide-cross-ligated nucleic acid (AmNA), and the like. Such modified DNA is also within the scope of "oligonucleotide," "oligonucleotide molecule," "polynucleotide," and "polynucleotide molecule" of the present disclosure.

As used herein, "ribonucleic acid (RNA)" means a molecule including at least one ribonucleotide monomer moiety. The "ribonucleotide" refers to a nucleotide that has a hydroxyl group at the 2'-position of the β-D-ribofuranose moiety. RNA may be modified with any functional group, and RNA variants include, but are not limited to, 2'-O-methyl RNA, 2' -fluoro-RNA, 2'-methoxyethyl-RNA (MOE), and the like. Such modified RNA is also within the scope of "oligonucleotide," "oligonucleotide molecule," "polynucleotide," and "polynucleotide molecule" of the present disclosure.

As used herein, "nucleobase" or "base" refers to a base component constituting a nucleic acid, and examples thereof include, but are not limited to, adenine (A), guanine (G), cytosine (C), thymine (T), uracil (U), hypoxanthine (inosine), 5-methylcytosine, N6-methyladenine, 1-methyladenine, pseudouracil, 1-methylpseudouracil, 5-bromouracil, 5-iodouracil, 6-thioadenine, 6-thioguanine, 4-thiouracil, 2-aminopurine, 2,6-diaminopurine, and the like.

As used herein, "template nucleic acid" refers to a nucleic acid having a sequence complementary to a polynucleotide or an oligonucleotide, or a variant thereof, to which the technique such as the production method of the present disclosure is applied.

As used herein, "3' end" of an oligonucleotide (or polynucleotide) or a variant thereof refers to a terminal monomer moiety attached to the 3' side of the oligonucleotide (or polynucleotide) or the variant thereof.

As used herein, "5' end" of an oligonucleotide (or polynucleotide) or a variant thereof refers to a terminal monomer moiety attached to the 5' side of the oligonucleotide (or polynucleotide) or the variant thereof.

As used herein, "ligation" means bonding via an arbitrary chemical bond, a linker, or the like, or an action of bonding, and the chemical bond is not limited thereto, and is usually a covalent bond.

As used herein, "conditions under which ligation occurs" refers to conditions under which an oligonucleotide (or polynucleotide) or a variant thereof can be ligated. In the present disclosure, as the "conditions under which ligation occurs", conditions using an enzyme having nucleic acid ligation activity such as ligase and chemical ligation conditions are assumed. Conditions under which chemical ligation occurs include temperature, pH, solvent, salt concentration (buffer, sodium chloride, etc.), and the like, and can be appropriately adjusted by those skilled in the art. Conditions for using an enzyme also include conditions suitable for generating a desired reaction catalyzed by the enzyme to be used, for example, concentration of a substrate such as a nucleic acid fragment, concentration of the enzyme, temp erature, pH, solvent, salt concentration (buffer, sodium chloride, etc.), and the like, and can be appropriately adjusted by those skilled in the art.

As used herein, "assembly" refers to a collection including the sequence of two or more fragment molecules of an oligonucleotide (or polynucleotide) or a variant thereof.

As used herein, a "splint" or "nucleic acid splint" is a nucleic acid molecule that is used interchangeably and has a sequence that, when used with another nucleic acid molecule, is complementary to a portion of the other nucleic acid molecule. The nucleic acid splint may be DNA or RNA, but is preferably DNA, in which case it is referred to as DNA splint.

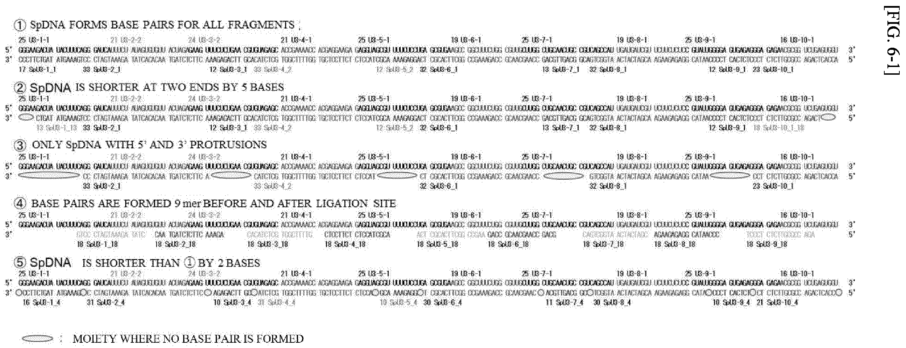

As used herein, "complementary" refers to a relationship between a region of one nucleic acid and a region of another nucleic acid, and refers to a relationship in which both can interact with a certain degree of affinity, and representatively means that a similar region of two single-stranded nucleic acids or a nucleotide sequence of two different regions of the same single-stranded nucleic acid has a nucleic acid base composition that enables hybridization. The sequences that hybridize with each other may be fully or partially complementary to the target sequence intended by standard nucleic acid base pairing (e.g., G:C, A:T, or A:U pairing). "Substantially complementary" may not be completely identical as long as hybridization proceeds, and in particular, in the case of the nucleic acid molecule of the present disclosure, both or either of the 5' end or the 3' end may be deleted. For example, it has been shown that the reaction proceeds without difficulty even if both of the 5' end and the 3' end are deficient by several bases (see Examples, FIGS. 6-1, 7-1, 8-1, etc.).

As used herein, "hybridize" refers to that nucleic acids form a complex in a complementary manner.

As used herein, "substantially complementary" refers to that the nucleic acid splint has a complementary sequence to the extent that the nucleic acid splint can form double strands with a single-stranded nucleic acid.

As used herein, the "joining point" refers to a moiety to which fragment molecules are ligated. In the present disclosure, a polynucleotide fragment molecule is ligated via a joining point to form a polynucleotide.

As used herein, "protrusion" refers to a portion where the 3' end of a nucleic acid extends beyond the 5' end of another complementary nucleic acid with which the nucleic acid hybridizes, or conversely, a portion where the 5' end of a nucleic acid extends beyond the 3' end of another complementary nucleic acid with which the nucleic acid hybridizes. In this case, it can be said that when two or more nucleic acid splints and the polynucleotide fragment molecule are aligned with a specific sequence, (preferably, the 3' end of the nucleic acid extends beyond the 5' end of the other complementary nucleic acid with which the nucleic acid hybridizes) the nucleic acid splints and the polynucleotide fragment molecule have a sequence designed to be shifted by one base or more (for example, 2 to 8 mer or the like).

As used herein, "align" (and alignment as a noun form thereof) refers to that a plurality of polynucleotide fragment molecules or nucleic acid splints are aligned so as to cause an interaction when polynucleotide fragment molecules and nucleic acid splints are hybridized, and usually refers to the alignment of complementary sequences to a certain extent. The alignment can also be executed by any software used in the art, for example, publicly available ones such as BLAST can be utilized, but the software that can be utilized is not limited thereto.

As used herein, "deletion" generally refers to the absence of a sequence that should be originally present, and in the context of the present disclosure, typically refers to the absence of a part of a base sequence (which may be one base) when a polynucleotide fragment molecule and a nucleic acid splint are hybridized.

As used herein, "there is substantially no deletion" generally refers to the presence of a sequence that should originally be present, or the presence of only a deletion at a level that does not hinder the achievement of the purpose even when there is no sequence, and in the context of the present disclosure, typically refers to a deletion of preferably 3 bases or less between nucleic acid splints with respect to the sequence of a single-stranded nucleic acid when the nucleic acid splints form double strands with the single-stranded nucleic acid. More preferably, 2 bases or less (e.g., 2 bases, 1 base, or 0 bases) are deleted, and even more preferably, 1 base or less is deleted. Here, it is not necessary for the nucleic acid splints to be complementary all the way to both ends of the single-stranded nucleic acid. When a plurality of nucleic acid splints is used, the plurality may not cover the corresponding specific sequence. That is, it is understood that when a plurality of nucleic acid splints is used, there may be deletion portions as compared to the corresponding specific sequence.

As used herein, "adjacent" refers to the positional relationship between two nucleic acids, and typically refers to that the positional relationship between one nucleic acid and another nucleic acid is parallel with a spacing of 2 bases or less (e.g., 2 bases, 1 base, or 0 bases).

As used herein, "distance" refers to a length between molecules in the context of the polynucleotide molecule or the like of the present disclosure, and is typically represented by the number of bases of a nucleic acid.

As used herein, "carrier" refers to a substance for immobilizing a nucleotide molecule when the nucleotide molecule is ligated. Examples of carrier include polystyrene, CPG (Controlled pore glass), magnetic beads, and the like, but are not limited thereto as long as a nucleotide molecule can be immobilized.

As used herein, "kit" refers to a unit in which a portion to be provided is usually provided in two or more compartments. The form of kit is preferred when the purpose is to provide compositions that, for reasons such as stability, should not be mixed at the time of provision but are preferably mixed immediately before use.

As used herein, "design" refers to providing a nucleic acid having a desired sequence and/or creating a schematic representation of such a nucleic acid.

In the context of the present description, "disease", "disorder", and "condition" are used interchangeably and refer to any condition in which a subject deviates from a normal condition (normal or healthy).

### (Preferred embodiments)

Preferred embodiments of the present disclosure will be described below. It is understood that the embodiments provided below are provided for a better understanding of the present disclosure, and that the scope of the present disclosure should not be limited to the following description. Therefore, it is apparent that those skilled in the art can appropriately make modifications within the scope of the present disclosure in view of the present description. It is also understood that the following embodiments of the present disclosure may be used alone or in combination.

### (Ligation of polynucleotides using nucleic acid splint)

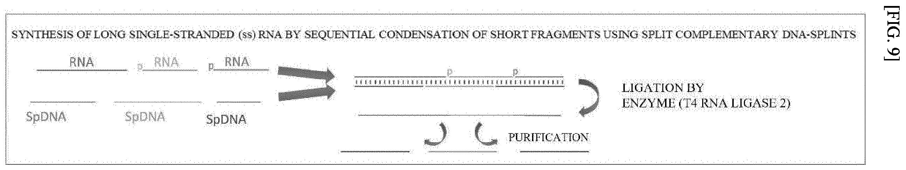

In one aspect, the present disclosure provides a method for producing a polynucleotide molecule having a specific sequence, the method including the steps of: 1) providing two or more polynucleotide fragment molecules including a portion of the specific sequence; and 2) ligating the two or more polynucleotide fragment molecules in the presence of two or more nucleic acid splints, in which each sequence of the two or more polynucleotide fragment molecules when assembled constitutes the specific sequence, the nucleic acid splints are substantially complementary to the polynucleotide molecule, and at least two of the nucleic acid splints when aligned with the specific sequence include a sequence which is substantially free of deletions. A schematic diagram of the present disclosure is shown in FIG. 9. As illustrated in the drawing, in one embodiment, the method of the present disclosure involves designing nucleic acid splints (here, DNA splints) to form strands which are complementary to the entire target sequence, which is the specific sequence. Then, unlike conventional techniques, this method is implemented by dividing the splints rather than using a single-stranded approach. As a result, the nucleic acid splints have significantly shorter strand lengths compared to single-stranded (ss) RNA, thereby facilitating purification. FIG. 10 is a schematic diagram.

As illustrated in FIG. 10, in one embodiment, the method of the present disclosure involves designing nucleic acid splints (here, DNA splints) to form strands which are complementary to the entire target sequence, which is the specific sequence. Then, unlike conventional techniques, this method is implemented by dividing the splints rather than using a single-stranded approach, and furthermore, preparing the divided splints in a non-continuous manner. As a result, the nucleic acid splints have significantly shorter strand lengths compared to single-stranded (ss) RNA, thereby facilitating purification. The method can also be used for RNA synthesized by in vitro transcription. By partially ligating synthetic oligo-nucleic acids including chemical modifications, it becomes possible to synthesize long nucleic acids having chemical modifications inserted at arbitrary positions.

In the present disclosure, a specific sequence can be any sequence desired for production or synthesis purposes. A part of the specific sequence may be any part as long as it is a part of the specific sequence for production or synthesis, and may be preferably at the 5' end or in the middle of the sequence. Once a specific sequence is specified, a polynucleotide fragment molecule including a part of the specific sequence can be prepared by synthesis or the like of a polynucleotide including the specific sequence.

In one preferred embodiment, the fragments used are 3 or more, 4 or more, 5 or more, 6 or more, 7 or more, 8 or more, 9 or more, or 10 or more. By using 3 or more fragments, the efficiency of the ligation reaction can be improved.

In the present disclosure, two or more polynucleotide fragment molecules prepared as described above are ligated in the presence of two or more nucleic acid splints. This ligation can be performed by any method, for example, using (enzymes such as ligase or polymerase, or under conditions that chemically promote ligation. Those skilled in the art can apply appropriate conditions. In the ligation step of the present disclosure, usually, each sequence of two or more polynucleotide fragment molecules is assembled to substantially constitute the specific sequence. Here, it is advantageous that the nucleic acid splint used is substantially complementary to the polynucleotide molecule and includes a sequence which is substantially free of deletions when at least two of the nucleic acid splints are aligned with the specific sequence.

According to the technique of the present disclosure, improvement of reactivity between polynucleotides and a high reaction yield can be achieved by using a technique of ligating polynucleotides using a nucleic acid splint. In addition, by using the technique of the present disclosure, a plurality of or all ligation reactions can be simultaneously started, and improvement of reactivity and a high reaction yield can be achieved.

In one embodiment, the polynucleotide fragment molecule used in the present disclosure is RNA or modified RNA. In the present embodiment, the modification may be halo (fluoro, chloro, iodide, and the like), substituted (including alkyl such as methyl, alkoxy such as methoxy and ethoxy, and the like) or unsubstituted alkyl (for example, methyl, ethyl, and the like), nucleic acid or modified nucleic acid (for example, BNA, LNA, and the like), phosphorothioate, etc., examples of other modifications are described elsewhere in the present description.

In one embodiment, the nucleic acid splint used in the present disclosure is DNA. The DNA splint may be modified.

In one embodiment, the polynucleotide fragment molecule used in the present disclosure is RNA or modified RNA, and the nucleic acid splint used in the present disclosure is DNA. In this case, an RNA fragment can be used as a ligation molecule, and a DNA splint can be used, and in this case, ligation is usually performed with an RNA ligase.

In a preferred embodiment, the RNA ligase used may include T4 RNA ligase, RtcB ligase, T4 DNA ligase (T4 DNA ligase having an RNA ligation action is known), Splint (registered trademark) Ligase, etc.

In one embodiment, the polynucleotide fragment molecule used in the present disclosure is monophosphorylated, more preferably a monophosphorylated RNA. The monophosphorylated RNA is advantageously used in a synthesis reaction using a ligase or the like. A monophosphate group may be at the 5' end or the 3' end or both. In one embodiment, the polynucleotide fragment molecule used in the present disclosure may not be in a phosphate form.

In one embodiment, the polynucleotide fragment molecule used in the present disclosure is provided by at least one method selected from a group consisting of chemical synthesis and transcriptional synthesis. The case of a polynucleotide fragment molecule provided by chemical synthesis is preferable because not only unmodified RNA or DNA but also modified RNA or DNA can be used. In certain embodiments, chemically synthesized or modified RNA can be used. Such chemically synthesized RNA or modified RNA may be realized using techniques known in the art (for example, without intending to be limiting, see Roy, S.; Caruthers, M. Synthesis of DNA/RNA and Their Analogs via Phosphoramidite and H-Phosphonate Chemistries. Molecules 2013, 18, 14268-14284. https://doi.org/10.3390/molecules181114268).

In another embodiment, the polynucleotide fragment molecule used in the present disclosure is provided by both the chemical synthesis method and the transcriptional synthesis method. The technique of the present disclosure can be used for both chemical synthesis and transcriptional synthesis, and can be used even when the two are mixed.

One specific embodiment includes providing the polynucleotide fragment molecule used in the present disclosure by transcriptional synthesis, and monophosphorylating the 5' end of the polynucleotide fragment molecule after the transcriptional synthesis, in which the ligation is performed using an RNA ligase. This is because the RNA ligase can be advantageously used by monophosphorylation. Monophosphorylation can be realized by using an enzyme such as RppH (RNA 5'pyrophosphohydrolase), but is not limited thereto.

In one embodiment, the nucleic acid splints used in the present disclosure are complementary to the polynucleotide molecules used in the present disclosure by 8 mer or more. Preferably, the nucleic acid splints are complementary by 9 mer or more, 10 mer or more, 11 mer or more, 12 mer or more, 13 mer or more, 14 mer or more, 15 mer or more, 16 mer or more, 17 mer or more, 18 mer or more, 19 mer or more, or 20 mer or more. The use of nucleic acid splints allows for easy removal through purification, and the nucleic acid splints can also be reused. It is observed that the efficiency of the dehydration condensation reaction by the enzyme is improved by having a certain complementary length.

**In** one embodiment, the distance between the nucleic acid splint aligned with the specific sequence used in the present disclosure and the nucleic acid splint adjacent to the nucleic acid splint aligned with the specific sequence used in the present disclosure is 2 bases or less. The nucleic acid splints are assembled with the fragment in a manner that the distance between the adjacent nucleic acid splints is 2 bases or less as compared with the case where the base pairing is only partially complementary, whereby the improvement of reactivity and the high reaction yield can be achieved. When the distance is appropriate, the improvement of reactivity and the higher reaction yield can be achieved, and the distance is preferably 2 bases or less, more preferably 1 base or less.

**In** one embodiment, the deletion used in the present disclosure is 2 bases or less, preferably 1 base or less. Without wishing to be bound by theory, the reason why it is preferable that the number of deletions is 1 or less is that the ligation activity is high when the number of deletions is small.

**In** one embodiment, the polynucleotide fragment molecule used in the present disclosure is 10 mer to 50 mer. 10 mer to 30 mer is preferable.

The length of the polynucleotide fragment molecule used in the present disclosure is not particularly limited. It is preferable that these polynucleotide fragment molecules have a length capable of forming a double strand with a nucleic acid splint, for example. Without wishing to be bound by theory, this is because the length capable of forming a double strand with the nucleic acid splint allows the reaction of the present disclosure to proceed efficiently. Those skilled in the art can appropriately set the length capable of forming a double strand with such a nucleic acid splint in consideration of the sequence information, the reaction conditions, and the like.

In one embodiment, the nucleic acid splint used in the present disclosure is 10 mer to 50 mer. 12 mer to 35 mer is preferable. By using a plurality of split nucleic acid splints rather than using long full-match single-stranded nucleic acid splints, the improvement of reactivity and the high reaction yield can be achieved.

The length of the nucleic acid splint used in the present disclosure is not particularly limited. It is preferable that these nucleic acid splints have a length capable of forming a double strand with a polynucleotide fragment molecule, for example.

In one embodiment, the nucleic acid splint used in the present disclosure has a sequence that protrudes from the joining point of the polynucleotide fragment molecule. The number of protruding bases may be any length as long as the technique of the present disclosure proceeds, and may be 1, 2, 3, 4, 5, 6, 7, 8, 9, 10 bases, or the like. The number of protruding bases may be any length as long as a complementary sequence between the nucleic acid splint and the polynucleotide fragment molecule is secured to an appropriate length (length as otherwise described As used herein, e.g., 8 mer, etc.). Preferably, the number of protruding bases is, for example, 1 to 15 mer, 1 to 10 mer, 2 to 8 mer, 3 to 8 mer, or the like, and may be, for example, 5 mer or more. Without wishing to be bound by theory, the reason why the protrusion is preferably 5 mer or more is that the ligation activity is high.

In one embodiment, the bases constituting the nucleotides of the present disclosure are usually the natural bases constituting a biologically existing polynucleotide molecule (for example, purine bases such as adenine and guanine, and pyrimidine bases such as cytosine, uracil, and thymine), but bases that do not constitute a biologically existing polynucleotide molecule (referred to in the present description as "non-natural base") may also be used in some cases. Examples of such non-natural bases include inosine, xanthine, hypoxanthine, nubularine, isoguanisine, and tubercidine, as well as variants of the bases described in the present disclosure.

Examples of the bases include, as natural bases, purine bases such as adenine and guanine, and pyrimidine bases such as cytosine, uracil, and thymine. In addition, examples of the bases include inosine, xanthine, hypoxanthine, nubularine, isoguanisine, and tubercidine. The aforementioned bases include, for example, alkyl derivatives such as 2-aminoadenine and 6-methylated purine; alkyl derivatives such as 2-propylated purine; 5-halouracil and 5-halocytosine; 5-propynyluracil and 5-propynylcytosine; 6-azouracil, 6-azocytosine, and 6-azothymine; 5-uracil (pseudouracil), 4-thiouracil, 5-halouracil, 5-(2-aminopropyl)uracil, and 5-aminoallyl uracil; 8-halogenated, aminated, thiolated, thioalkylated, hydroxylated, and other 8-substituted purines; 5-trifluoromethylated and other 5-substituted pyrimidines; 7-methylguanine; 5-substituted pyrimidines; 6-azapyrimidine; N-2, N-6, and O-6 substituted purines (including 2-aminopropyladenine); 5-propynyluracil and 5-propynylcytosine; dihydrouracil; 3-deaza-5-azacytosine; 2-aminopurine; 5-alkyluracil; 7-alkylguanine; 5-alkylcytosine; 7-deazaadenine; N6,N6-dimethyladenine; 2,6-diaminopurine; 5-amino-allyl-uracil; N3-methyluracil; substituted 1,2,4-triazoles; 2-pyridone; 5-nitroindole; 3-nitropyrrole; 5-methoxyuracil; uracil-5-oxyacetic acid; 5-methoxycarbonylmethyluracil; 5-methyl-2-thiouracil; 5-methoxycarbonylmethyl-2-thiouracil; 5-methylaminomethyl-2-thiouracil; 3-(3-amino-3-carboxypropyl)uracil; 3-methylcytosine; 5-methylcytosine; N4-acetylcytosine; 2-thiocytosine; N6-methyladenine; N6-isopentenyladenine; 2-methylthio-N6-isopentenyladenine; N-methylguanine; and O-alkylated bases, among others. Also included are purine and pyrimidine bases, for example, those disclosed in US 3,687,808, "Concise Encyclopedia Of Polymer Science And Engineering", p. 858 to 859, edited by Kroschwitz J. I., John Wiley & Sons, 1990, and Englisch et al., Angewandte Chemie, International Edition, 1991, Vol.30, p.613.

In one embodiment, at least one end of the polynucleotide fragment molecule used in the present disclosure has an association with a carrier. Preferably, the carrier used in the present disclosure is polystyrene, CPG (Controlled pore glass), or magnetic beads. By utilizing the carrier, the yield can be increased.

In one embodiment, the polynucleotide ligation reaction used in the present disclosure is started simultaneously at a plurality of locations. Without wishing to be bound by theory, the reasons why co-initiation is preferred include the simplification of the ligation reaction step, but are not limited thereto.

In one embodiment, the polynucleotide ligation reaction used in the present disclosure is started sequentially. Without wishing to be bound by theory, the reason why sequential start is preferred is that the yield may be higher when the reaction is performed with sequential start.

In a preferred embodiment, it is more preferable that a method for producing a polynucleotide molecule having the specific sequence of the present disclosure is characterized by performing:
1) a step of providing two or more polynucleotide fragment molecules including a part of the specific sequence, where three or more fragment molecules are provided, and
2) a step of ligating two or more polynucleotide fragment molecules in the presence of two or more nucleic acid splints, where three or more nucleic acid splints are provided, and the ligation is performed by dehydration condensation or the like, in which
when the sequences of two or more polynucleotide fragment molecules are assembled, the sequences constitute the specific sequence, the nucleic acid splints are complementary to the polynucleotide molecules, and at least two of the nucleic acid splints, when aligned with the specific sequence, include a sequence which is free of deletions.

In one embodiment, the ligation is performed via enzymatic ligation reactions. In this case, the enzymaticligation reactions are performed simultaneously or at different times. Preferably, the enzymatic ligation reactions are performed simultaneously. It has been confirmed that the reactions proceed without difficulty even at different times, and the present invention is not particularly limited. In the enzymatic ligation reactions, (A) one ligation reaction is performed in a combination that results in a total extension which is shorter than the specific sequence of the polynucleotide fragment molecule, then the next ligation reaction is performed using a product of the one ligation reaction, and (B) (A) may be repeated as necessary. The combination in which the total extension is 175 to 200 mer may have any length, for example, 100 to 500 mer, 150 to 400 mer, or 175 to 200 mer, 50 to 300 mer, 80 to 500 mer, 200 to 400 mer, or the like.

The technique according to the present disclosure is considered to have the following features. As one non-limiting feature, by starting all the enzymatic ligation reactions simultaneously, for example, in the synthesis of a long sequence (for example, 560 mer), it is more efficient to start the reactions by a reaction of a shorter sequence (for example, 175 to 200 maer), and it is confirmed that the reaction product can be confirmed even when the reactions are started simultaneously. In addition, as a non-limiting feature, by designing a nucleic acid splint (for example, DNA splint) in a manner that all the bases included in the target sequence form base pairs, and designing the ligation site to protrude by several bases by using a nucleic acid splint (for example, DNA splint) divided in several places instead of simply using a long full-match single-stranded nucleic acid splint (for example, DNA splint), synthesis of long (500 mer or more, or the like) single-stranded RNA is successfully provided by an unprecedented combination of solid phase synthesis and ligation, for example.

Therefore, in the present disclosure, as a non-limiting feature, it is understood that the efficiency of the enzymatic ligation reactions of the present disclosure is better as compared with the case where the nucleic acid splint (for example, DNA splint) is assembled to the ligation site ± several bases, and the efficiency of the enzymatic ligation reactions is better as compared with the case where a long full-match single-stranded nucleic acid splint (for example, DNA splint) is simply used. In addition, as a non-limiting feature, it is understood to be preferable that the reaction efficiency is better when the nucleic acid splint (for example, DNA splint) is assembled to all the fragments, as compared with the case where some base pairing is deleted. Furthermore, although the present disclosure is not limited to this, as a typical example, the strand length of the nucleic acid splint (e.g., DNA splint) is shorter than the final product, which allows for easy removal through purification. It is understood that reuse of the nucleic acid splint (for example, DNA splint) is also possible in principle. As a non-limiting feature of the present disclosure, for example, when a single-stranded long target sequence nucleic acid (RNA or the like) is synthesized by continuous synthesis, a carrier having a high loading amount such as polystyrene cannot be used, but the carrier can be used for a short target sequence nucleic acid (RNA or the like), and an increase in yield per batch of RNA synthesis can be expected.

In one aspect, the present disclosure provides polynucleotides ligated by the ligation reactions described in the present description. Without wishing to be bound by theory, this polynucleotide has a characteristic of being a long polynucleotide using a plurality of chemically synthesized short polynucleotides as a starting substance, and may be one that could not be conventionally provided.

### (Production kit)

In another aspect, the present disclosure provides a kit for producing a polynucleotide molecule having the specific sequence described As used herein, the kit including: 1) two or more polynucleotide fragment molecules including a portion of the specific sequence described in the present description; and 2) two or more nucleic acid splints, in which each sequence of the two or more polynucleotide fragment molecules when assembled constitutes the specific sequence described As used herein, the nucleic acid splints described in the present description are substantially complementary to the polynucleotide molecule, and at least two of the nucleic acid splints include a sequence which is substantially free of deletions when aligned with the specific sequence. It is understood that the various embodiments described here can be applied by appropriately combining any of the forms described in (Ligation of polynucleotides using nucleic acid splint).

In one preferred embodiment, the fragments used are 3 or more, 4 or more, 5 or more, 6 or more, 7 or more, 8 or more, 9 or more, or 10 or more. By using 3 or more fragments, the efficiency of the ligation reaction can be improved. Without wishing to be limited, the polynucleotide fragment molecules used may be, but are not limited to, 100 or less, 90 or less, 80 or less, 70 or less, 60 or less, 50 or less, 40 or less, or the like, and a greater number of polynucleotide fragment molecules may be used.

In one embodiment, a kit is provided in which the nucleic acid splints used in the kit of the present disclosure are complementary to the polynucleotide molecules used in the present disclosure by 8 mer or more. Preferably, the nucleic acid splints are complementary by 9 mer or more, 10 mer or more, 11 mer or more, 12 mer or more, 13 mer or more, 14 mer or more, 15 mer or more, 16 mer or more, 17 mer or more, 18 mer or more, 19 mer or more, or 20 mer or more. The use of nucleic acid splints allows for easy removal through purification as the nucleic acid splints are shorter than the final product, and the nucleic acid splints can also be reused. When arranged in a kit, these nucleic acid splints may include those actually used, as well as spare ones, those with slightly different sequences, and those with different lengths. Those skilled in the art can select an appropriate nucleic acid splint in use to implement the methods of the present disclosure.

In one embodiment, in the kit of the present disclosure, the distance between the nucleic acid splint aligned with the specific sequence and the nucleic acid splint adjacent to the nucleic acid splint aligned with the specific sequence used in the present disclosure is 2 bases or less. The nucleic acid splints are assembled with the fragment in a manner that the distance between the adjacent nucleic acid splints is 2 bases or less as compared with the case where the base pairing is only partially complementary, whereby the improvement of reactivity and the high reaction yield can be achieved. When arranged in a kit, these nucleic acid splints may include those actually used, as well as spare ones, those with slightly different sequences, and those with different lengths. Those skilled in the art can select an appropriate nucleic acid splint in use to implement the methods of the present disclosure.

In one embodiment, the nucleic acid splint used in the kit of the present disclosure has a deletion of two bases or less when aligned with the specific sequence.

In one embodiment, the polynucleotide fragment molecules used in the kit of the present disclosure are 14 mer to 46 mer. 10 mer to 30 mer is preferable. By using a plurality of split nucleic acid splints rather than using long full-match single-stranded nucleic acid splints, the improvement of reactivity and the high reaction yield can be achieved. When arranged in a kit, these polynucleotide fragment molecules may include those actually used, as well as spare ones, those with slightly different sequences, and those with different lengths. Those skilled in the art can select an appropriate polynucleotide fragment molecule in use to implement the methods of the present disclosure.

In one embodiment, the nucleic acid splint used in the kit of the present disclosure has a sequence that protrudes by 4 mer or more from the joining point of the polynucleotide fragment molecule. When arranged in a kit, these nucleic acid splints and polynucleotide fragment molecules may include those actually used, as well as spare ones, those with slightly different sequences, and those with different lengths, and may be labeled to facilitate finding appropriate combinations. Those skilled in the art can select an appropriate polynucleotide fragment molecule in use to implement the methods of the present disclosure.

In one embodiment, at least one end of the polynucleotide fragment molecule included in the kit of the present disclosure has an association with a carrier. Preferably, the carrier included in the kit of the present disclosure is polystyrene, CPG (Controlled pore glass), or magnetic beads. By utilizing such a carrier, the yield can be increased.

### (Material provision / design program)

When arranged in a kit, these carriers may include those actually used, as well as spare ones and those of different kinds, and may be labeled to facilitate finding appropriate combinations. Those skilled in the art can select an appropriate carrier in use to implement the methods of the present disclosure.

In another aspect, the present disclosure provides a method for producing a polynucleotide molecule having the specific sequence described As used herein, the method including the steps of: A) designing two or more polynucleotide fragment molecules including a portion of a desired sequence based on the specific sequence; B) designing two or more nucleic acid splints based on the specific sequence; C) providing the polynucleotide fragment molecules and the nucleic acid splints which have been designed; and D) ligating the polynucleotide fragment molecules which have been designed in the presence of the nucleic acid splints which have been designed, in which each sequence of the two or more polynucleotide fragment molecules when assembled constitutes the specific sequence, the nucleic acid splints are substantially complementary to the polynucleotide molecule, and at least two of the nucleic acid splints include a sequence which is substantially free of deletions when aligned with the specific sequence. It is understood that the various embodiments described here can be applied by appropriately combining any of the forms described in (Ligation of polynucleotides using nucleic acid splint).

In another aspect, the present disclosure provides a method for producing a polypeptide fragment and a nucleic acid splint for producing a polynucleotide molecule having the specific sequence described As used herein, the method including the steps of: A) designing two or more polynucleotide fragment molecules including a portion of the desired sequence based on the specific sequence; B) designing two or more nucleic acid splints based on the specific sequence; and C) producing the polynucleotide fragment molecules and the two or more nucleic acid splints which have been designed, in which each sequence of the two or more polynucleotide fragment molecules when assembled constitutes the specific sequence, the nucleic acid splints are substantially complementary to the polynucleotide molecule, and at least two of the nucleic acid splints include a sequence which is substantially free of deletions when aligned with the specific sequence. It is understood that the various embodiments described here can be applied by appropriately combining any of the forms described in (Ligation of polynucleotides using nucleic acid splint).

In another aspect, the present disclosure provides a method for designing a polypeptide fragment and a nucleic acid splint for producing a polynucleotide molecule having the specific sequence, the method including the steps of: A) designing two or more polynucleotide fragment molecules including a portion of a desired sequence based on the specific sequence; and B) designing two or more nucleic acid splints based on the specific sequence, in which each sequence of the two or more polynucleotide fragment molecules when assembled constitutes the specific sequence, the nucleic acid splints are substantially complementary to the polynucleotide molecule, and at least two of the nucleic acid splints include a sequence which is substantially free of deletions when aligned with the specific sequence. It is understood that the various embodiments described here can be applied by appropriately combining any of the forms described in (Ligation of polynucleotides using nucleic acid splint).

In another aspect, the present disclosure provides a program for causing a computer to execute a method of designing a polypeptide fragment and a nucleic acid splint for producing a polynucleotide molecule having the specific sequence, the method including the steps of: A) designing two or more polynucleotide fragment molecules including a portion of a desired sequence based on the specific sequence; and B) designing two or more nucleic acid splints based on the specific sequence, in which each sequence of the two or more polynucleotide fragment molecules when assembled constitutes the specific sequence, the nucleic acid splints are substantially complementary to the polynucleotide molecule, and at least two of the nucleic acid splints include a sequence which is substantially free of deletions when aligned with the specific sequence. It is understood that the various embodiments described here can be applied by appropriately combining any of the forms described in (Ligation of polynucleotides using nucleic acid splint).

### (Pharmaceutical and other applications)

The present disclosure can be applied in pharmaceuticals.

For example, the compositions and methods of the present disclosure can be utilized to treat an individual in need thereof. In certain embodiments, the individual is a mammal, such as a human, or a non-human mammal. When administered to an animal, such as a human, the composition or compound is preferably administered as a pharmaceutical composition including, for example, a compound of the present disclosure and a pharmaceutically acceptable carrier. Pharmaceutically acceptable carriers are well known in the art and include, for example, aqueous solutions such as water or buffered saline, or other solvents or vehicles such as glycols, glycerol, oils such as olive oil, or organic esters for injection. In a preferred embodiment, when such a pharmaceutical composition is for administration to a human, particularly in a case of administration by an invasive route (that is, for example, routes such as injection or implantation that avoid transport or diffusion through the epithelial barrier), the aqueous solution is pyrogen-free or substantially pyrogen-free. An excipient can be selected, for example, to carry out a delayed release of the agent or to selectively target one or a plurality of cells, tissues or organs. The pharmaceutical composition can be in unit dosage forms, such as tablets, capsules (including sprinkle capsules and gelatin capsules), granules, freeze-dried preparations for reconstitution, powders, liquids, syrups, suppositories, or injections. The composition can also be present in a transdermal delivery system, e.g., a skin patch. The compositions can also be present in liquids suitable for topical administration, such as eye drops.

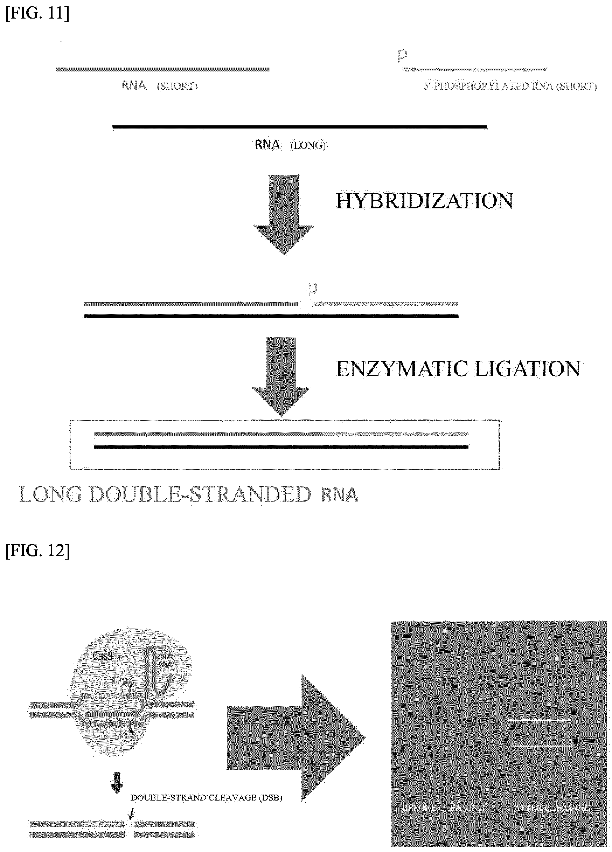

The present disclosure can also be widely utilized in genome editing technology. Although not limited, the present invention can be applied to, for example, sgRNAs used in genome editing, particularly, production of sgRNAs (J. Filippova et al., Biochimie 167 2019 49-60, Ayal Hendel et al. nature biotechnology 33 2015 985-989). Without wishing to be bound by theory, since the activity seems to be increased by using an oligonucleotide including a modified (varied) base of the present disclosure or a variant thereof (oligo-nucleic acid), it is conceivable as an application that fragments of an oligo-nucleic acid including a modified base synthesized using the technique of the present disclosure are connected and produced. As another embodiment, it is also conceivable to make relatively long double strands as described in T. Seya et al. Advanced Drug Delivery Reviews 147 2019 37-43 such that the strands serve as templates for each other without using template DNA (FIG. 11).

Another embodiment may include, for example, long non-coding RNA (lncRNA) or mRNA as the target.

In using polynucleotide molecules produced by the present disclosure as a pharmaceutical, "subjects" to which administration is contemplated include, but are not limited to, humans (i.e. male or female subjects of any age group, e.g. pediatric subjects (for example, toddlers, children, and adolescence) or adult subjects (for example, a young adult, a middle-aged adult, or an elderly adult)) and/or other non-human animals, such as mammals (e.g., a primate (e.g. cynomolgus monkey, rhesus monkey), a commercially relevant mammal, e.g., a cow, a pig, a horse, a sheep, a goat, a cat, and/or a dog) and birds (for example, commercially relevant birds such as chickens, ducks, geese, and/or turkeys), reptiles, amphibians, and fish. In certain embodiments, the non-human animal is a mammal. The non-human animal may be male or female at any time of development. The non-human animal may be a transgenic animal.

In the context of the present description, unless specifically limited, the terms "treat," "treating," and "treatment" refer to actions that occur while the subject is afflicted with a disease, disorder, or condition, and aim to reduce the severity of or delay or slow the progression of the disease, disorder, or condition (narrow sense of "therapy"). The terms also encompass actions taken before the subject becomes afflicted with the disorder to inhibit the occurrence of the disease, disorder, or condition or to reduce the severity once the subject has actually developed the disease, disorder, or condition ("prevention").

In general, an "effective amount" of a compound refers to an amount sufficient to elicit the desired biological response, e.g., to treat a disease, disorder, or condition. It will be readily understood by those skilled in the art that the effective amount of the compound of the present disclosure may vary depending on factors such as the desired biological endpoint, the pharmacokinetics of the compound, the disease, disorder, or condition to be treated, the mode of administration, as well as the age, the health condition, and the subject. The effective amount encompasses treatment and prophylactic therapy.

In the context of the present description, unless specifically limited, a "therapeutically effective amount" of a compound refers to an amount sufficient to provide a therapeutic effect in the treatment of a disease, disorder or condition, or to delay or minimize one or more symptoms associated with the disease, disorder or condition. A therapeutically effective amount of a compound means an amount of a therapeutic agent that, alone or in combination with other therapies, provides a therapeutic effect in the treatment of a disease, disorder, or condition. The term "therapeutically effective amount" may encompass an amount that improves overall therapy, reduces or prevents the symptoms or causes of a disease, disorder, or condition, or enhances the therapeutic efficacy of another therapeutic agent.

In the context of the present description, unless specifically limited, a "prophylactically effective amount" of a compound is an amount sufficient to prevent a disease, disorder or condition or one or more symptoms associated with the disease, disorder or condition, or to prevent the recurrence thereof. A prophylactically effective amount of a compound means an amount of a therapeutic agent that, alone or in combination with other agents, provides a prophylactic effect in the prevention of a disease, disorder, or condition. The term "prophylactically effective amount may encompass an amount that improves overall prophylaxis or enhances the prophylactic efficacy of another prophylactic agent.

Exemplary diseases, disorders, or conditions include those characterized by proliferative, neurological, immune, endocrine, cardiovascular, hematological, inflammatory, and cell death-related disorders, but are not limited thereto.

In the context of the present description, a proliferative disease, disorder or condition includes, but is not limited to, cancer, hematologic tumors, proliferative breast diseases, proliferative lung disorders, proliferative colonic disorders, proliferative liver disorders, and proliferative ovarian disorders.

Oligonucleotides or variants thereof described in the present description can be used in nucleic acid medicaments.

### (Method for producing compound of present disclosure)

Hereinafter, specific synthesis examples of the method for producing the compound of the present disclosure are given in Examples, but the method is not limited thereto, and representative schemes will be described below, but the present disclosure is by no means limited thereto.

The compound of the present disclosure, although not limited these, can be produced, for example, by the production methods described below. These production methods can be appropriately improved on the basis of knowledge of a person skilled in organic synthetic chemistry. In the following production methods, as the compound used as a raw material, a salt thereof may be used as long as it does not interfere with the reaction.

The polynucleotide fragment molecule and the nucleic acid splint of the present disclosure can be prepared, for example, by a solid phase synthesis method. More specifically, the polynucleotide fragment molecule and the nucleic acid splint of the present disclosure can be prepared using a nucleic acid synthesizer (nS-8II manufactured by GeneDesign Inc.; Oligopilot manufactured by Cytiva) based on a phosphoramidite method. The phosphoramidite method is a method in which three steps of deblocking, coupling, and oxidation are taken as one cycle and this cycle is repeated until a desired base sequence is obtained. For each reagent, the phosphoramidite method can be performed, for example, using porous glass or polystyrene as a solid phase carrier, a dichloroacetic acid toluene solution or a trichloroacetic acid dichloromethane solution as a deblocking solution, 5-benzylthio-1H-tetrazole as a coupling agent, an iodine solution as an oxidizing agent, and an acetic anhydride solution and an N-methylimidazole solution as capping solutions. For the cutting out and deprotection from the solid phase carrier after the solid phase synthesis, for example, an aqueous ammonia solution, a mixture of an aqueous ammonia solution and ethanol, and a mixture of methylamine and an aqueous ammonia solution are added to perform the deprotection of the base part and the phosphate group and the cutting out from the solid phase carrier, then the solid phase carrier is filtered, and then the 2'-hydroxyl group is deprotected using tetrabutylammonium fluoride or hydrofluoric acid neutralized with triethylamine to prepare polynucleotide fragment molecules and nucleic acid splints.

The amidites used in the solid phase synthesis method are not particularly limited. For example, amidites with the following protecting groups in the structural formula (I) below can be used: R1 protected by a tert-butyldimethylsilyl (TBDMS) group, bis(2-acetoxy)methyl (ACE) group, (triisopropylsilyloxy)methyl (TO M) group, (2-cyanoethoxy)ethyl (CEE) group, (2-cyanoethoxy)methyl (CEM) group, p-tolylsulfonylethoxymethyl (TEM) group, (2-cyanoethoxy)methoxymethyl (EMM) group, etc. Specific examples include: TBDMS amidites (TBDMS RNAAmidites, product name, ChemGenes Corporation), ACE amidites, TOM amidites, CEE amidites, CEM amidites, TEM amidites (as reviewed by Chakhmakhcheva: Protective Groups in the Chemical Synthesis of Oligoribonucleotides, Russian Journal of Bioorganic Chemistry, 2013, Vol. 39, No. 1, pp. 1-21), EMM amidites (as described in WO 2013/027843 A), and others.

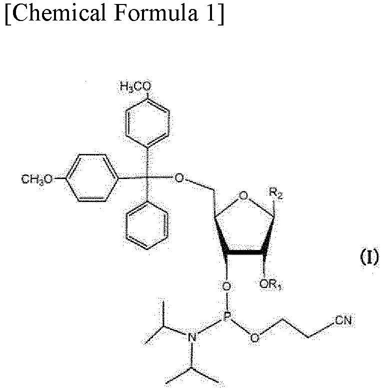

The in vitro transcription reaction is generally a reaction in which a single-stranded RNA is synthesized using a double-stranded DNA having a promoter region sequence specifically recognized by the enzyme as a template using T7 RNA polymerase or SP6 RNA polymerase (for example, see Beckert B, Masquida B. Synthesis of RNA by in vitro transcription. Methods Mol Biol. 2011;703:29-41. doi: 10.1007/978-1-59745-248-9_3. PMID: 21125481.).

The present disclosure has been described above with reference to preferred embodiments for easy understanding. Hereinafter, the present disclosure will be described based on Examples, but the above description and the following Examples are provided for the purpose of illustration only and not for the purpose of limiting the present disclosure. Accordingly, the scope of the present disclosure is not limited to the embodiments or Examples specifically described As used herein, but is limited only by the claims. Examples

In the present Examples, Examples related to production and use of the compound of the present disclosure will be described.

### (Example 1) Tracking of ligation reaction when filling with nucleic acid strands without gap

In the present Example, nucleic acid strands were filled without gaps to perform a ligation reaction. Details are as follows.

### (Materials and procedure)

The following reagents and fragments were used.
· Reagents
T4 RNA Ligase 2 (manufactured by New England Biolab Inc., 10 U/µL)
1 M Tris-HCl (pH 7.5) (manufactured by NIPPON GENE CO., LTD.)
1 M MgCl₂ (manufactured by NIPPON GENE CO., LTD.)
100 mM ATP (manufactured by FUJIFILM Wako Pure Chemical Corporation)
1 M DTT (manufactured by Sigma-Aldrich Co. LLC)
· Nucleic acid fragment
· Nucleic acid strand

### (Common)

1. Sg1 5'-GGAAAUUAGGUGCGCUUGGC -3' (SEQ ID NO: 1)
2. sg2 5'-pGUUUUAGAGCUAGAA -3' (SEQ ID NO: 2)
3. sg3 5'-pAUAGCAAGUUAAAAUAAGG -3' (SEQ ID NO: 3)
4. sg4 5'-pCUAGUCCGUUAUCAACUU -3' (SEQ ID NO: 4)
5. sg5 5'-pGAAAAAGUGGCACC -3' (SEQ ID NO: 5)
6. sg6 5'-pGAGUCGGUGCUUUU -3' (SEQ ID NO: 6)

### (Novel method)

7. Spsg1 5'-ctaaaacgccaagcgcacctaatttcc-3' (SEQ ID NO: 7)
8. Spsg2 5'-ttttaacttgctatttctagct-3' (SEQ ID NO: 8)
9. Spsg3 5'-ctttttcaagttgataacggactagcctta-3' (SEQ ID NO: 9)
10. Spsg4 5'-aaaagcaccgactcggtgcca-3' (SEQ ID NO: 10)

### (Conventional Method 1) 11. Spsg1_16 5'-tctaaaacgccaagcg-3' (SEQ ID NO: 11)

12. Spsg2_14 5'-ttgctatttctagc-3' (SEQ ID NO: 12)
13. Spsg3_18 5'-acggactagccttatttt-3' (SEQ ID NO: 13)
14. Spsg4_14 5'-ctttttcaagttga-3' (SEQ ID NO: 14)
15. Spsg5_14 5'-ccgactcggtgcca-3' (SEQ ID NO: 15)

### (Conventional Method 2)

16. Spsg100 5'-aaaagcaccgactcggtgccactttttcaagttgataacggactagccttattttaacttgctatttctagctctaaaacgccaagcgca cctaatttcc-3' (SEQ ID NO: 16)
The nucleic acid combinations used are shown in FIG. 1-1.

Enzymatic ligation was performed according to the following procedure.

### (Method of the present disclosure)

In this example, split complementary strand nucleic acid splints were assembled into all RNAs.

Details of the procedure are as follows.
(1) The nucleic acid strands 1 to 6 were mixed so as to have a final concentration of 25 µM each and the nucleic acid strands 7 to 10 were mixed so as to have a final concentration of 37.5 µM each, then the mixture was heated at 80°C for 3 minutes, and then cooled to 5°C over 25 minutes.
(2) The nucleic acid strands and the reaction reagents were mixed at the following concentrations.

10 µM nucleic acid strands 1 to 6
15 µM nucleic acid strands 7 to 10

### [The solution of (1) was diluted 2.5 times]

50 mM Tris-HCl (pH7.5)
2 mM MgCl₂
0.4 mM ATP
1 mM DTT
0.5 U/µL T4 RNA Ligase 2
(3) The solution mixed in (2) was subjected to a ligation reaction at 25°C.
(4) The ligation reaction solution was appropriately sampled 1, 4, 24, and 48 hours after the start of the reaction, mixed with an equal amount of 5 mM EDTA solution, and used for subsequent analysis.

### (Comparative Example 1: Conventional Method 1)

Complementary strand nucleic acid splints were assembled from the joining point several bases at a time.

Details of the procedure are as follows.

### (Technique)

(1) The nucleic acid strands 1 to 6 were mixed so as to have a final concentration of 25 µM each and the nucleic acid strands 11 to 15 were mixed so as to have a final concentration of 37.5 µM each, then the mixture was heated at 80°C for 3 minutes, and then cooled to 5°C over 25 minutes.
(2) The nucleic acid strands and the reaction reagents were mixed at the following concentrations.

10 µM nucleic acid strands 1 to 6
15 µM nucleic acid strands 11 to 15

### [The solution of (1) was diluted 2.5 times.]

50 mM Tris-HCl (pH7.5)
2 mM MgCl₂
0.4 mM ATP
1 mM DTT
0.5 U/µL T4 RNA Ligase 2

(3) The solution mixed in (2) was subjected to a ligation reaction at 25°C.

(4) The ligation reaction solution was appropriately sampled 1, 4, 24, and 48 hours after the start of the reaction, mixed with an equal amount of 5 mM EDTA solution, and used for subsequent analysis.

### (Comparative Example 2: Conventional Method 2)

Substantially the same conditions as in the above Example were used except that a long single-stranded complementary nucleic acid splint was used.

### (Technique)

Details of the procedure are as follows.
(1) The nucleic acid strands 1 to 6 were mixed so as to have a final concentration of 25 µM each and the nucleic acid strand 16 was mixed so as to have a final concentration of 37.5 µM, then the mixture was heated at 80°C for 3 minutes, and then cooled to 5°C over 25 minutes.
(2) The nucleic acid strands and the reaction reagents were mixed at the following concentrations.

10 µM nucleic acid strands 1 to 6
15 µM nucleic acid strand 16

### [The solution of (1) was diluted 2.5 times.]

50 mM Tris-HCl (pH7.5)
2 mM MgCl₂
0.4 mM ATP
1 mM DTT
0.5 U/µL T4 RNA Ligase 2

(3) The solution mixed in (2) was subjected to a ligation reaction at 25°C.

(4) The ligation reaction solution was appropriately sampled 1, 4, 24, and 48 hours after the start of the reaction, mixed with an equal amount of 5 mM EDTA solution, and used for subsequent analysis.

### (Denatured-PAGE)

Details of the procedure are as follows.
(1) 1 µL of the enzymatic ligation reaction sample was mixed with bromophenol blue-containing formamide and heated at 80°C for 5 minutes. The heated sample was quenched immediately in ice.
(2) 2 µL of the solution of (1) was applied to an 8% acrylamide gel containing 7.5M urea, 1xTBE, and electrophoresed using 1xTBE buffer at a voltage of 300 V for 20 minutes.
(3) The gel after electrophoresis was stained with ethidium bromide, and then photographed with a transilluminator.

### (Measurement by UHPLC/LC-MS)

Details of the procedure are as follows.

The enzymatic ligation reaction solution from which the enzyme protein had been removed by phenol/chloroform extraction or the like was subjected to UHPLC/LC-MS analysis under the following conditions.
· Column: ACQUITY UPLC Oligonucleotide BEH C18 Column, 130 A, 1.7 µm, 2.1 mm x 100 mm
· Mobile phase: A) 100mM hexafluoro-2-propanol (HFIP)-8mM triethylamine (TEA), B) methanol (MeOH)
· Analysis conditions: B5-20%, 60 minutes, 80°C, 0.2 ml/min

### (Reaction tracking by UHPLC)

Details of the procedure are as follows.

UHPLC analysis was performed under the following conditions.
· Column: ACQUITY UPLC Oligonucleotide BEH C18 Column, 130 A, 1.7 µm, 2.1 mm x 100 mm
· Mobile phase: A) 100mM hexafluoro-2-propanol (HFIP)-8mM triethylamine (TEA), B) methanol (MeOH)
· Analysis conditions: B5-20%, 60 minutes, 80°C, 0.2 ml/min

### (Results)

The results of enzymatic ligation with ssRNA (short RNA) 100 mer 6-split RNA ligase 2 are shown in FIG. 1-2.

The results of Denatured-PAGE are shown in FIG. 1.

As shown in FIG. 1-2, it was revealed that in the novel method, a 100 mer ligation reaction product was generated in a shorter time than in Conventional Method 1.

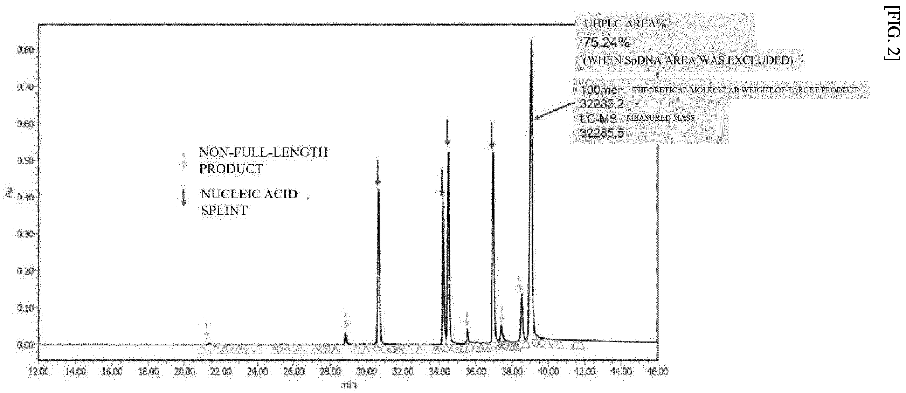

The results of measurement by HPLC/LC-MS are shown in FIG. 2.

The theoretical molecular weight of the target product in this test is 32285.2. The measured mass in LC-MS analysis was 32285.5. Therefore, it was revealed that the enzymatic ligation product in this test was a target 100 mer RNA.

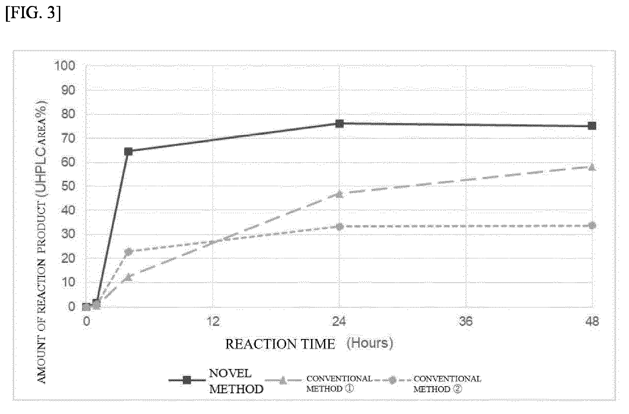

Next, the results of tracking the reaction by UHPLC are shown in Table 1 and FIG. 3.

The UHPLC area% of the 100 mer enzymatic ligation product in this test is shown in FIG. 3. The reaction rate was higher when the ligation reaction was performed by the novel method than in Conventional Methods 1 and 2.

**[Table 1]**

| Amount of reaction product (UHPLC area %) | | | |
|---|---|---|---|
| | Novel method | Conventional method 1 | Conventional method 2 |
| 0h | 0 | 0 | 0 |
| 1h | 1.79 | 0.62 | 1 |
| 4h | 64.69 | 12. 38 | 22. 88 |
| 24h | 76.14 | 46.91 | 33.21 |
| 48h | 75.24 | 58.24 | 33.57 |

### (Example 2) Example using modified base (PS modification, 2'-OMe, 2'-F, etc.)

Next, a similar ligation experiment was performed using a modified base (PS modification, 2'-OMe, 2'-F, etc.).

The procedure will be described below.

### (Materials)

### Oligo-nucleic acids used

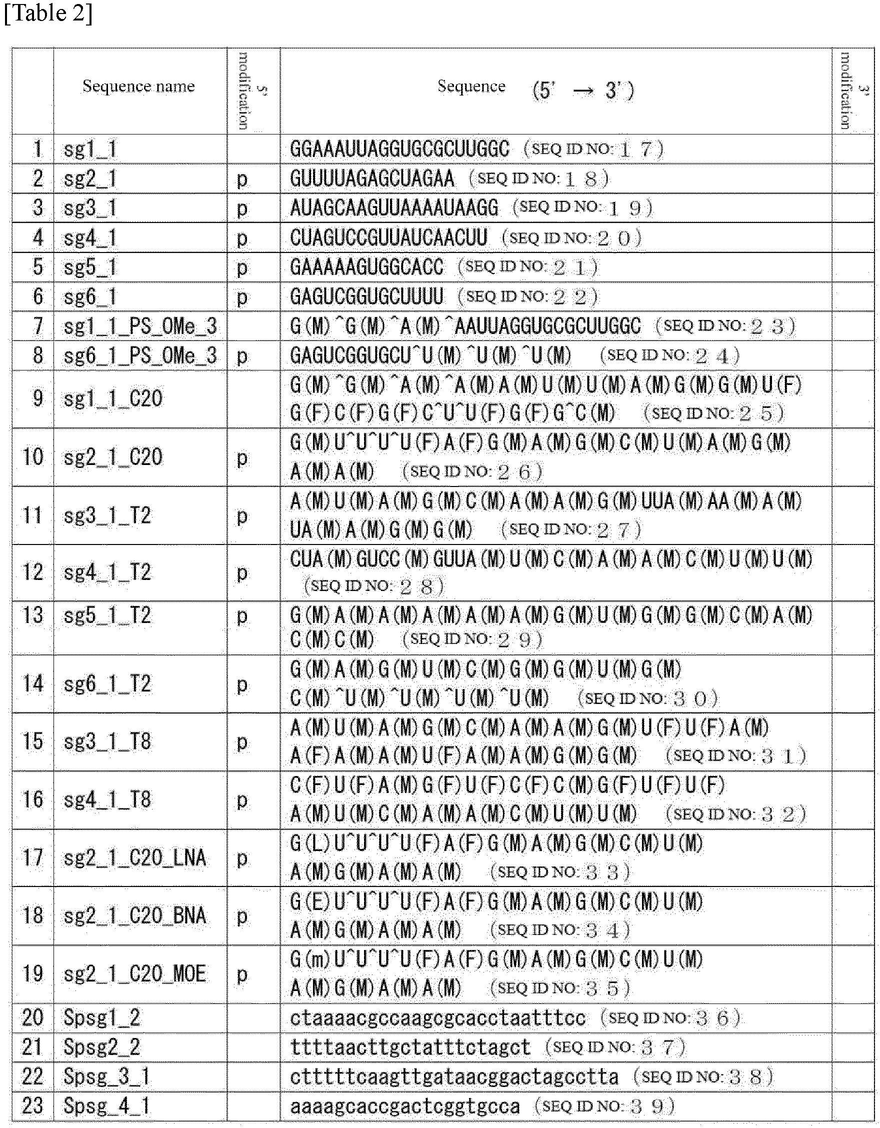

Sequence representation: Upper Case =RNA/N(M) = 2'-OMe RNA/N(F) = 2'-F RNA/N(L) = LNA/N(E) = BNANC-NMe/N(m) = 2'-MOE / p = Phosphorylation /^ = Phosphorothioated

The nucleic acid combination in the ligation reaction was performed as follows.

**[Table 3]**

| | | RNA fragments used |
|---|---|---|
| | Natural | 1, 2, 3, 4, 5, 6 |
| Subject to PS modification and 2'-OMe modification of end | | 2, 3, 4, 5, 7, 8 |
| | Highly modified 1 | 9, 10, 11, 12, 13, 14 |
| | Highly modified 2 | 9, 10, 13, 14, 15, 16 |
| | Highly modified 3 - containing LNA | 9, 11, 12, 13, 14, 17 |
| Highly modified 4 - containing BNA-NC (N-Me) | | 9, 11, 12, 13, 14, 18 |
| Highly modified 5 - containing 2'-MOE | | 9, 11, 12, 13, 14, 19 |

The sequence after the ligation reaction is as follows.

**[Table 4-1]**

| | Sequence after reaction |
|---|---|
| Natural | GGAAAUUAGGUGCGCUUGGCGUUUUAGAGCUAGAAAUAGCAAGUUAAAAU AAGGCUAGUCCGUUAUCAACUUGAAAAAGUGGCACCGAGUCGGUGCUUUU (SEQ ID NO. 40) |
| Subject to PS modification and 2'-OMe modification of end | G(M)^G(M)^A(M)^AAUUAGGUGCGCUUGGCGUUUUAGAGCUAGAAAUAGCAA GUUAAAAUAAGGCUAGUCCGUUAUCAACUUGAAAAAGUGGCACCGAGUCG GUGCU^U(M)^U(M)^U(M) (SEQ ID NO: 41) |

**[Table 4-2]**

| | |
|---|---|
| Highly modified 1 | G(M)^G(M)^A(M)^A(M)A(M)U(M)U(M)A(M)G(M)G(M)U(F)G(F)C(F)G(F)C^U^U(F) G(F)G^C(M)G(M)U^U^U^U(F)A(F)G(M)A(M)G(M)C(M)U(M)A(M)G(M)A(M)A(M) A(M)U(M)A(M)G(M)C(M)A(M)A(M)G(M)UUA(M)AA(M)A(M)UA(M)A(M)G(M)G(M) CUA(M)GUCC(M)GUUA(M)U(M)C(M)A(M)A(M)C(M)U(M)U(M)G(M)A(M)A(M)A(M) A(M)A(M)G(M)U(M)G(M)G(M)C(M)A(M)C(M)C(M)G(M)A(M)G(M)U(M)C(M)G(M) G(M)U(M)G(M)C(M)^U(M)^U(M)^U(M)^U(M)   (SEQ ID NO: 42) |
| Highly modified 2 | G(M)^G(M)^A(M)^A(M)A(M)U(M)U(M)A(M)G(M)G(M)U(F)G(F)C(F)G(F)C^U^U(F) G(F)G^C(M)G(M)U^U^U^U(F)A(F)G(M)A(M)G(M)C(M)U(M)A(M)G(M)A(M)A(M) A(M)U(M)A(M)G(M)C(M)A(M)A(M)G(M)U(F)U(F)A(M)A(F)A(M)A(M)U(F)A(M)A(M) G(M)G(M)C(F)U(F)A(M)G(F)U(F)C(F)C(M)G(F)U(F)U(F)A(M)U(M)C(M)A(M)A(M) C(M)U(M)U(M)G(M)A(M)A(M)A(M)A(M)A(M)G(M)U(M)G(M)G(M)C(M)A(M)C(M) C(M)G(M)A(M)G(M)U(M)C(M)G(M)G(M)U(M)G(M)C(M)^U(M)^U(M)^U(M)^U(M) (SEQ ID NO: 43) |
| Highly modified 3 containing LNA | G(M)^G(M)^A(M)^A(M)A(M)U(M)U(M)A(M)G(M)G(M)U(F)G(F)C(F)G(F)C^U^U(F) G(F)G^C(M)G(L)U^U^U^U(F)A(F)G(M)A(M)G(M)C(M)U(M)A(M)G(M)A(M)A(M)CU A(M)GUCC(M)GUUA(M)U(M)C(M)A(M)A(M)C(M)U(M)U(M)G(M)A(M)A(M)A(M)A(M) A(M)G(M)U(M)G(M)G(M)C(M)A(M)C(M)C(M)G(M)A(M)G(M)U(M)C(M)G(M)G(M)U(M) G(M)C(M)^U(M)^U(M)^U(M)^U(M)A(M)U(M)A(M)G(M)C(M)A(M)A(M)G(M)UUA(M)A A(M)A(M)UA(M)A(M)G(M)G(M) (SEQ ID NO: 44) |
| Highly modified 4 containing BNA-NC (N-Me) | G(M)^G(M)^A(M)^A(M)A(M)U(M)U(M)A(M)G(M)G(M)U(F)G(F)C(F)G(F)C^U^U(F)G(F) G^C(M)G(E)U^U^U^U(F)A(F)G(M)A(M)G(M)C(M)U(M)A(M)G(M)A(M)A(M)CUA(M)GU CC(M)GUUA(M)U(M)C(M)A(M)A(M)C(M)U(M)U(M)G(M)A(M)A(M)A(M)A(M)G(M) U(M)G(M)G(M)C(M)A(M)C(M)C(M)G(M)A(M)G(M)U(M)C(M)G(M)G(M)U(M)G(M) C(M)^U(M)^U(M)^U(M)^U(M)A(M)U(M)A(M)G(M)C(M)A(M)A(M)G(M)UUA(M)AA(M) A(M)UA(M)A(M)G(M)G(M) (SEQ ID NO: 45) |
| Highly modified 5 containing 2'-MOE | G(M)^G(M)^A(M)^A(M)A(M)U(M)U(M)A(M)G(M)G(M)U(F)G(F)C(F)G(F)C^U^U(F)G(F) G^C(M)G(m)U^U^U^U(F)A(F)G(M)A(M)G(M)C(M)U(M)A(M)G(M)A(M)A(M)CUA(M)GU CC(M)GUUA(M)U(M)C(M)A(M)A(M)C(M)U(M)U(M)G(M)A(M)A(M)A(M)A(M)G(M) U(M)G(M)G(M)C(M)A(M)C(M)C(M)G(M)A(M)G(M)U(M)C(M)G(M)G(M)U(M)G(M) C(M)^U(M)^U(M)^U(M)^U(M)A(M)U(M)A(M)G(M)C(M)A(M)A(M)G(M)UUA(M)AA(M) A(M)UA(M)A(M)G(M)G(M) (SEQ ID NO: 4 6) |

### (Procedure)

sgRNA in which about 3 to 5 bases at 5'- and 3'- ends were 2'-O-methylated (Hendel et al., Nat Biotechnol, 33;985-989,2015): there is no report of highly modified sgRNA, but it was shown that highly modified sgRNA can be synthesized with reference to, for example, a modified two-split guide RNA (Mir et al., Nat Commun, 9; 2641, 2018).

Details of the procedure are as follows.
(1) The nucleic acid strands 1 to 19 described in Table 2 were mixed in the combination of Table 3 in a manner that the final concentrations were each 25 µM and the 20 to 23 final concentrations were each 37.5 µM, and the mixture was heated at 80°C for 3 minutes and then cooled to 5°C over 25 minutes.
(2) The nucleic acid strands and the reaction reagents were mixed at the following concentrations.

10 µM nucleic acid strands 1 to 19 (combination of Table 3)
15 µM nucleic acid strands 20 to 23

### [The solution of (1) was diluted 2.5 times]

50 mM Tris-HCl (pH7.5)
2 mM MgCl₂
0.4 mM ATP
1 mM DTT
0.5 U/µL T4 RNA Ligase 2

(3) The solution mixed in (2) was subjected to a ligation reaction at 25°C.

(4) The ligation reaction solution was appropriately sampled 1, 4, 24, and 48 hours after the start of the reaction, mixed with an equal amount of 5 mM EDTA solution, and used for subsequent analysis.

### (Results)

The reaction product 48 hours after the start of the reaction was subjected to LC-MS analysis.

The results are as follows.

**[Table 5]**

| | | Theoretical molecular weight | Measured value (after deconvolution) |
|---|---|---|---|
| | Natural | 32285.12 | 32283.9 |
| Subject to PS modification and 2'-OMe modification of end | | 32465.68 | 32264.3 |
| | Highly modified 1 | 33547.87 | 33546.0 |
| | Highly modified 2 | 33571.77 | 33570.8 |
| | Highly modified 3 - containing LNA | 33545.86 | 33544.6 |
| Highly modified 4 - containing BNA-NC (N-Me) | | 33574.90 | 33573.6 |
| Highly modified 5 - containing 2'-MOE | | 33591.93 | 33590.8 |

From the above, it was shown that ligation can be performed by this method even in a long nucleic acid including a modified nucleic acid).

The RNA synthesized by this method can be used, for example, for cleaving and editing genomic DNA by the CRISPR-Cas9 method. Specifically, the 100 mer RNA synthesized by the present method and double-stranded DNA having any strand length targeted by the 20 bases at the 5' end are mixed with Cas9 protein in an appropriate buffer, and the DNA cleavage activity can be examined. RNA-Cas9 complexes can also be administered to cultured cells and in vivo using appropriate DDS to edit genomic DNA.

### (FIG. 12).

### (Example 4) Enzymatic ligation with ssRNA 220 mer 10-split RNA ligase 2

Next, enzymatic ligation with ssRNA 220 mer 10-split RNA ligase 2 was performed.

Details are as follows.

### (Materials)

Except for the used nucleic acid fragments shown in Table 6 below, the same materials as in Example 1 were used.

### (Procedure)

Except for the used nucleic acid fragments shown in Table 6 below, the same materials as in Example 1 were used.

**[Table 6]**

| | | |
|---|---|---|
| U3-1_1 | | GGGAAGACUAUACUUUCAGGGAUCA (SEQ ID NO:47 ) |
| U3-2_2 | p | UUUCUAUAGUGUGUUACUAGA (SEQ ID NO: 48 ) |
| U3-3.2 | p | GAAGUUUCUCUGAACGUGUAGAGC (SEQ ID NO: 49 ) |
| U3-4_1 | p | ACCGAAAACCACGAGGAAGAG (SEQ ID NO: 50) |
| U3-5_1 | p | AGGUAGCGUUUUCUCCUGAGCGUGA (SEQ ID NO: 51) |
| U3-6_1 | p | AGCCGGCUUUCUGGCGUUG (SEQ ID NO:52) |
| U3-7_1 | p | CUUGGCUGCAACUGCCGUCAGCCAU (SEQ ID NO:53) |
| U3-8_1 | p | UGAUGAUCGUUCUUCUCUC (SEQ ID NO:54) |
| U3-9_1 | p | CGUAUUGGGGAGUGAGAGGGAGAGA (SEQ ID NO:55) |
| U3-10-1 | p | ACGCGGUCUGAGUGGU (SEQ ID NO:56) |

**[Table 7]**

| | | |
|---|---|---|
| SpU3-1_1 | | tgaaagtatagtcttccc (SEQ ID NO:57) |
| SpU3-2_1 | | acttctctagtaacacactatagaaatgatccc (SEQ ID NO:58) |
| SpU3-3_1 | | acgttcagagaa (SEQ ID NO:59) |
| SpU3-4_2 | | tacctctcttcctcgtggttttcggtgctctac (SEQ ID NO:60) |
| SpU3-5_2 | | aggagaaaacge (SEQ ID NO:61) |
| SpU3-6_1 | | ccaagcaacgccagaaagccggcttcacgctc (SEQ ID NO:62) |
| SpU3-7_1 | | acggcagttgcag (SEQ ID NO:63) |
| SpU3-8_1 | | aatacggagagaagaacgatcatcaatggctg (SEQ ID NO:64) |
| SpU3-9_1 | | ctctcactcccc (SEQ ID NO:65) |
| SpU3-10_1 | | accactcagaccgcgttctctcc (SEQ ID NO:66) |

### (Results)

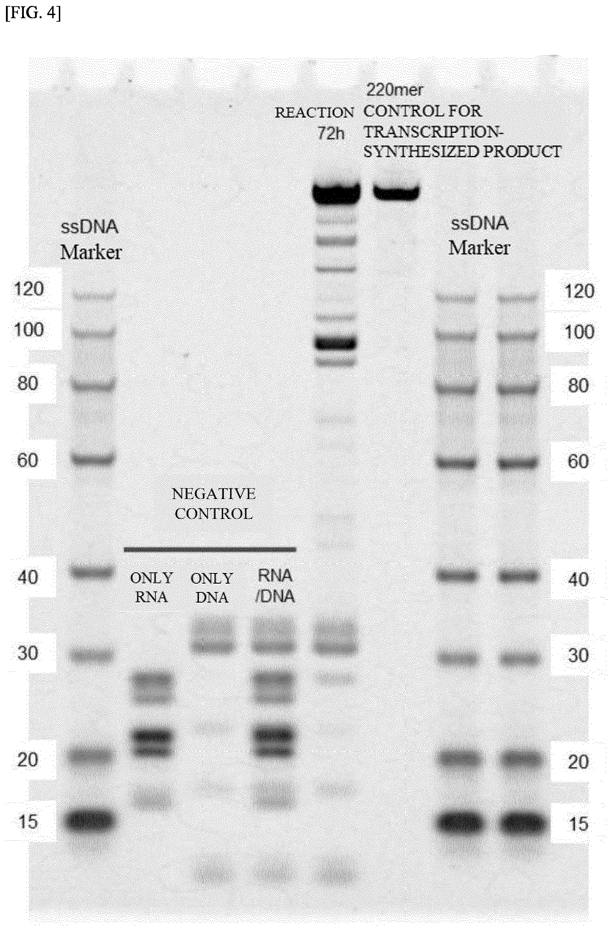

FIG. 4 shows the results of Denatured-PAGE.

10-split fragments were subjected to an enzymatic ligation reaction. The product after 72h of reaction was subjected to Denatured-PAGE analysis. As a control, in-vitro transcripts of identical sequence to the ligation product were analyzed simultaneously. The results are shown in FIG. 4. An enzymatic ligation product that seems to be identical to the 220 mer transcript was confirmed.

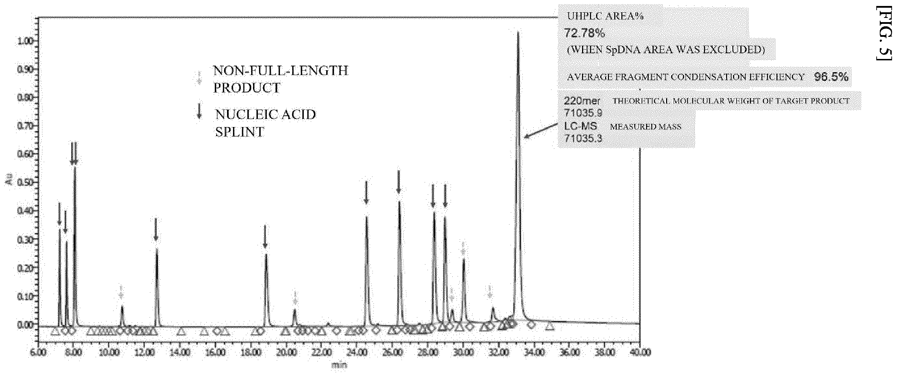

Next, the UHPLC/LC-MS results are shown in FIG. 5.

The theoretical molecular weight of the target product in this test is 71035.9. The measured mass in LC-MS analysis was 71035.3. Therefore, it was revealed that the enzymatic ligation product in this test was a target 220 mer RNA.

### (Example 5) Experiment with various sequences

Next, experiments with various sequences were performed. Experiments were performed using sequences as shown in Table 3. (Some are from the above Examples).

Details are as follows.

### (Materials)

The same as Example 4 except for the nucleic acid splints used shown in Table 8.

**[Table 8]**

| ① | | |
|---|---|---|
| SpU3-1_1 | | tgaaagtatagtcttccc (SEQ ID NO:67) |
| SpU3-2_1 | | acttctctagtaacacactatagaaatgatccc (SEQ ID NO:68) |
| SpU3-3_1 | | acgttcagagaa (SEQ ID NO:69) |
| SpU3-4_2 | | tacctctcttcctcgtggttttcggtgctctac (SEQ ID NO:70) |
| SpU3-5_2 | | aggagaaaacgc (SEQ ID NO:71) |
| SpU3-6_1 | | ccaagcaacgccagaaagccggcttcacgctc (SEQ ID NO:72) |
| SpU3-7_1 | | acggcagttgcag (SEQ ID NO:73) |
| SpU3-8_1 | | aatacggagagaagaacgatcatcaatggctg (SEQ ID NO:74) |
| SpU3-9_1 | | ctctcactcccc (SEQ ID NO:75) |
| SpU3-10_1 | | accactcagaccgcgttctctcc (SEQ ID NO:76) |

**[Table 9]**

| ② | | |
|---|---|---|
| SpU3-1_13 | | tgaaagtatagtc (SEQ ID NO:77) |
| SpU3-2_1 | | acttctctagtaacacactatagaaatgatccc (SEQ ID NO:78) |
| SpU3-3_1 | | acgttcagagaa (SEQ ID NO:79) |
| SpU3-4_2 | | tacctctcttcctcgtggttttcggtgctctac (SEQ ID NO:80) |
| SpU3-5_2 | | aggagaaaacgc (SEQ ID NO:81) |
| SpU3-6_1 | | ccaagcaacgccagaaagccggcttcacgctc (SEQ ID NO:82) |
| SpU3-7_1 | | acggcagttgcag (SEQ ID NO:8 3) |
| SpU3-8_1 | | aatacggagagaagaacgatcatcaatggctg (SEQ ID NO:84) |
| SpU3-9_1 | | ctctcactcccc (SEQ ID NO:85) |
| SpU3-10_18 | | tcagaccgcgttctctcc (SEQ ID NO:86) |

**[Table 10]**

| ③ | | |
|---|---|---|
| SpU3-2_1 | | acttctctagtaacacactatagaaatgatccc (SEQ ID NO:87) |
| SpU3-4_2 | | tacctctcttcctcgtggttttcggtgctctac (SEQ ID NO:88) |
| SpU3-6_1 | | ccaagcaacgccagaaagccggcttcacgctc (SEQ ID NO:89) |
| SpU3-8_1 | | aatacggagagaagaacgatcatcaatggctg (SEQ ID NO:90) |
| SpU3-10_1 | | accactcagaccgcgttctctcc (SEQ ID NO:91) |

**[Table 11]**

| ④ | | |
|---|---|---|
| SpU3-1_18 | | ctatagaaatgatccctg (SEQ ID NO:92) |
| SpU3-2_18 | | agaaacttctctagtaac (SEQ ID NO:93) |
| SpU3-3_18 | | gttttcggtgctctacac (SEQ ID NO:94) |
| SpU3-4_18 | | acgctacctctcttcctc (SEQ ID NO:95) |
| SpU3-5_18 | | aagccggcttcacgctca (SEQ ID NO:96) |
| SpU3-6_18 | | gcagccaagcaacgccag (SEQ ID NO:97) |
| SpU3-7_18 | | cgatcatcaatggctgac (SEQ ID NO:98) |
| SpU3-8_18 | | cccaatacggagagaaga (SEQ ID NO:99) |
| SpU3-9_18 | | agaccgcgttctctccct (SEQ ID NO:100) |

**[Table 12]**

| ⑤ | | |
|---|---|---|
| SpU3-1_4 | | gaaagtatagtcttcc (SEQ ID NO:101) |
| SpU3-2_4 | | cttctctagtaacacactatagaaatgatcc (SEQ ID NO:102) |
| SpU3-3_4 | | cgttcagaga (SEQ ID NO:103) |
| SpU3-4_4 | | acctctcttcctcgtggttttcggtgctcta (SEQ ID NO:104) |
| SpU3-5_4 | | ggagaaaacg (SEQ ID NO:105) |
| SpU3-6_4 | | caagcaacgccagaaagccggcttcacgct (SEQ ID NO:106) |
| SpU3-7_4 | | cggcagttgca (SEQ ID NO:107) |
| SpU3-8_4 | | atacggagagaagaacgatcatcaatggct (SEQ ID NO:108) |
| SpU3-9_4 | | tctcactccc (SEQ ID NO:109) |
| SpU3-10_4 | | ccactcagaccgcgttctctc (SEQ ID NO:110) |

### (Procedure)

The same as Example 4 except for the nucleic acid splints used shown in Tables 8 to 12.

1 used the same nucleic acid splints as in Example 4 (the nucleic acid splints form base pairs for all fragments).
2 shortened the strand lengths of the nucleic acid splints at 5' and 3' end sides by 5 bases.
3 used only the nucleic acid splints having long strand lengths of 23 to 33 mer among the nucleic acid splints of 1.
4 formed base pairs 9 mer before and after the ligation site.

In 5, both ends of the nucleic acid splint are shorter by one base.

The sequences used are shown in FIG. 6-1.

### (Results)

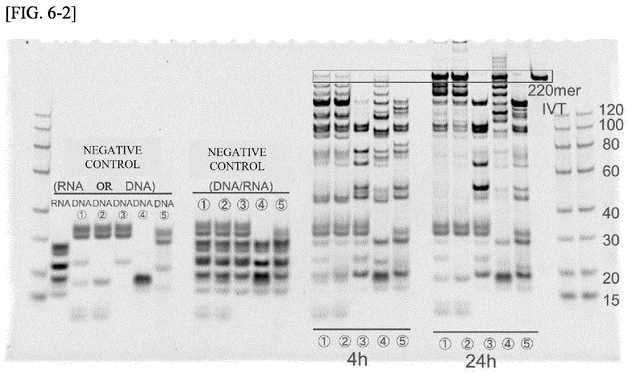

FIG. 6-2 shows the results of Denatured-PAGE.

The results of Denatured-PAGE of the enzymatic ligation product in Example 5 are shown. Compared to 1, the amount of full-length product did not change in the test of 2. That is, it was shown that the ligation reaction proceeds equally even if both ends are short with respect to the full length of the nucleic acid splint. In the test of 3, the amount of full-length product significantly decreased. It was also shown that the nucleic acid splints not involved in the joining point are important for the ligation reaction. In the test of 4, the amount of full-length reaction product slightly decreased. In addition, the side reaction product having an anti-molecular weight increased. In the test of 5, the amount of full-length product significantly decreased. It was suggested that when there is a gap in the formation of double strands between the nucleic acid splints and the RNA fragments, the reaction hardly proceeds.

### (Example 6) Enzymatic ligation with ssRNA 560 mer 28-split RNA ligase 2

Next, enzymatic ligation with ssRNA 560 mer 28-split RNA ligase 2 was performed. Sequence information is shown in Tables 13 and 14.

Details are as follows.

### (Materials)

**[Table 13]**

| | | |
|---|---|---|
| NL-1_1 | | GGCAAUCCGGUACUGUUGGUAAAGC (SEQ ID NO:11 1) |
| NL-2_1 | p | CACCAUGGUCUUCAC (SEQ ID NO:112) |
| NL-3_1 | p | ACUCGAAGAUUUCGUUGGGGACUGG (SEQ ID NO:11 3) |
| NL-4_1 | p | CGACAGACAGCCGGC (SEQ ID NO:114) |
| NL-5_2 | p | UACAACCUGGACCAAGUCCUUGAACAG (SEQ ID NO:1 1 5) |
| NL-6_2 | p | GGAGGUGUGUCCA (SEQ ID NO:116) |
| NL-7_1 | p | GUUUGUUUCAGAAUCUCGGGGUGUC (SEQ ID NO: 1 1 7) |
| NL-8_1 | p | CGUAACUCCGAUCCA (SEQ ID NO:118) |
| NL-9_1 | p | AAGGAUUGUCCUGAGCGGUGAAAAUG (SEQ ID NO:1 1 9) |
| NL-10_1 | p | GGCUGAAGAUCGAC (SEQ ID NO:120) |
| NL-11_4 | p | AUCCAUGUCAUCAUCCCGUAUGAAGG (SEQ ID NO:1 2 1) |
| NL-12_4 | p | UCUGAGCGGCGACCA (SEQ ID NO:122) |
| NL-13_2 | p | AAUGGGCCAGAUCGAAAAAAUUUUUAAGGU (SEQ ID NO:123) |
| NL-14_2 | p | GGUGUACCCUGU (SEQ ID NO:124) |
| NL-15_2 | p | GGAUGAUCAUCACUUUAAG (SEQ ID NO:125) |
| NL-16_2 | p | GUGAUCCUGCACUAUGGC (SEQ ID NO:126) |
| NL-17_1 | p | ACACUGGUAAUCGACGGGGUUACGC (SEQ ID NO:12 7) |
| NL-18_1 | p | CGAACAUGAUCGACU (SEQ ID NO:128) |
| NL-19_1 | p | AUUUCGGACGGCCGUAUGAAGGCAU (SEQ ID NO:12 9) |
| NL-20_1 | p | CGCCGUGUUCGACG (SEQ ID NO:130) |
| NL-21_1 | p | GCAAAAAGAUCAGUGUAACAGGGA (SEQ ID NO:13 1) |
| NL-22_1 | p | CCCUGUGGAACGGCAA (SEQ ID NO:132) |
| NL-23_1 | p | CAAAAUUAUCGACGAGCGCCUGAUCA (SEQ ID NO:1 33) |
| NL-24_1 | p | ACCCCGACGGCUCCCU (SEQ ID NO:134) |
| NL-25_1 | p | GCUGUUCCGAGUAACCAUCAACGG (SEQ ID NO:13 5) |
| NL-26_1 | p | AGUGACCGGCUGG (SEQ ID NO:136) |
| NL-27_1 | p | CGGCUGUGCGAACGCAUUCUGGCGUAA (SEQ ID NO:1 37) |
| NL-28_1 | p | UUCUAGAGUCGGGGC (SEQ ID NO:138) |

**[Table 14]**

| | | |
|---|---|---|
| SpNL-1_1 | | accaacagtaccggattgcc (SEQ ID NO:139) |
| SpNL-2_1 | | cgagtgtgaagaccatggtggettt (SEQ ID NO:14 0) |
| SpNL-3_1 | | ccccaacgaaatctt (SEQ ID NO:141) |
| SpNL-4_1 | | gttgtagccggctgtctgtcgccagt (SEQ ID NO:1 42) |
| SpNL-5_1 | | aggacttggtccag (SEQ ID NO:143) |
| SpNL-6_1 | | caaactggacacacctccctgttca (SEQ ID NO:14 4) |
| SpNL-7_1 | | cccgagattctgaaa (SEQ ID NO:145) |
| SpNL-8_1 | | tcctttggatcggagttacggacac (SEQ ID NO:14 6) |
| SpNL-9_1 | | accgctcaggacaa (SEQ ID NO:147) |
| SpNL-10_1 | | tggatgtcgatcttcagcccattttc (SEQ ID NO:1 48) |
| SpNL-11_2 | | cgggatgatgaca (SEQ ID NO: 149) |
| SpNL-12_2 | | ccatttggtcgccgctcagaccttcata (SEQ ID NO: 150) |
| SpNL-13_1 | | aaaattttttcgatctggc (SEQ ID NO: 151) |
| SpNL-14_2 | | catccacagggtacaccacctta (SEQ ID NO:15 2) |
| SpNL-15_2 | | atcaccttaaagtgatgat (SEQ ID NO:153) |
| SpNL-16_2 | | agtgtgccatagtgcagg (SEQ ID NO:154) |
| SpNL-17_1 | | accccgtcgattacc (SEQ ID NO:155) |
| SpNL-18_1 | | cgaaatagtcgatcatgttcggcgta (SEQ ID NO:1 56) |
| SpNL-19_1 | | ttcatacggccgtc (SEQ ID NO:157) |
| SpNL-20_1 | | ttttgccgtcgaacacggcgatgcc (SEQ ID NO:15 8) |
| SpNL-21_1 | | gttacagtgatct (SEQ ID NO:159) |
| SpNL-22_1 | | attttgttgccgttccacagggtccct (SEQ ID NO:1 60) |
| SpNL-23_1 | | aggcgctcgtcgata (SEQ ID NO:161) |
| SpNL-24_1 | | acagcagggagccgtcggggttgatc (SEQ ID NO:1 62) |
| SpNL-25_1 | | gatggttactcgga (SEQ ID NO:163) |
| SpNL-26_1 | | agccgccagccggtcactccgtt (SEQ ID NO:16 4) |
| SpNL-27_1 | | ccagaatgcgttcgcac (SEQ ID NO:165) |
| SpNL-28_1 | | gccccgactctagaattacg (SEQ ID NO:166) |

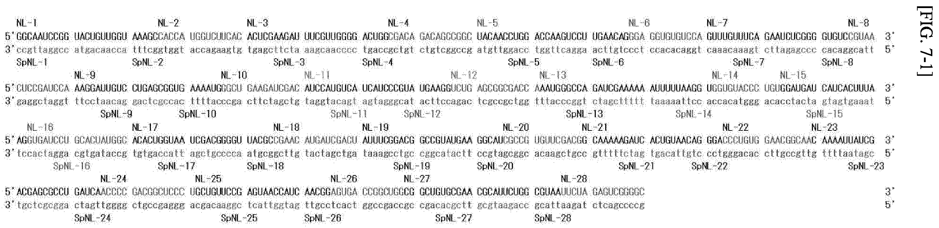

The sequences used are shown in FIG. 7-1.

### (Procedure)

(1) The RNA strands and the nucleic acid splints were mixed, then heated at 80°C for 3 minutes, and then cooled to 5°C over 25 minutes.
(2) The nucleic acid strands and the reaction reagents were mixed at the following concentrations.
   1 (560 mer in total)
   1 µM RNA nucleic acid strands 1-28 28 kinds
   1.5 µM nucleic acid splints 1-28 28 kinds
   50 mM Tris-HCl (pH7.5)
   2 mM MgCl₂
   0.4 mM ATP
   1 mM DTT
   5% PEG8000
   0.5 U/µL T4 RNA Ligase 2
   2_1-10 (200 mer in total)
   2.8 µM RNA nucleic acid strands 1-10 10 kinds
   4.2 µM nucleic acid splints 1-10 10 kinds
   50 mM Tris-HCl (pH7.5)
   2 mM MgCl₂
   0.4 mM ATP
   1 mM DTT
   5% PEG8000
   0.5 U/µL T4 RNA Ligase 2
   2_11-19 (185 mer in total)
   3.1 µM RNA nucleic acid strands 11-19 9 kinds
   4.7 M nucleic acid splints 11-19 9 kinds
   50 mM Tris-HCl (pH7.5)
   2 mM MgCl₂
   0.4 mM ATP
   1 mM DTT
   5% PEG8000
   0.5 U/µL T4 RNA Ligase 2
   2_20-28 (175 mer in total)
   3.1 µM RNA nucleic acid strands 20-28 9 kinds
   4.7 M nucleic acid splints 20-28 9 kinds
   50 mM Tris-HCl (pH7.5)
   2 mM MgCl₂
   0.4mM ATP
   1 mM DTT
   5% PEG8000
   0.5 U/µL T4 RNA Ligase 2
(3) The solution mixed in (2) was subjected to a ligation reaction at 25°C.
(4) The reaction solution of 2 was mixed so as to have the same composition as that of 1 and the reaction was continued 16 hours after the start of the reaction. The scheme is shown in Table 4.

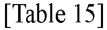

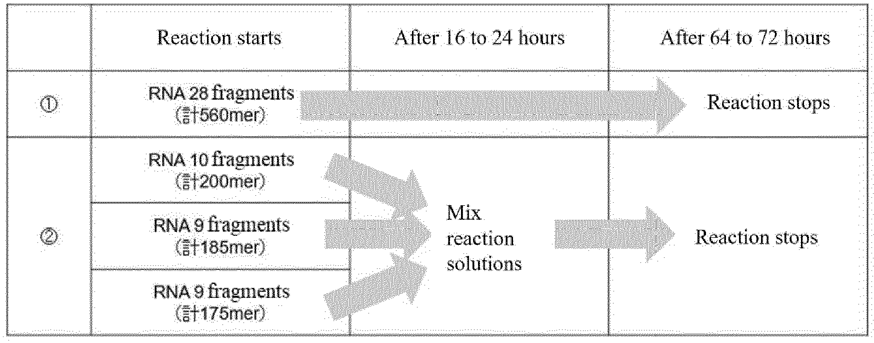

### (In vitro translation)

(1) The purified ligation product was subjected to in vitro translation. PUREfrex (registered trademark) 2.0 (manufactured by GeneFrontier Corporation) was used, and the method involved mixing the reagents and RNA according to the method recommended by the manufacturer.
(2) The mixture was reacted at 37°C for 6 hours.
(3) Post-reaction products were stored at -30°C and used for subsequent assays.

### (Luciferase assay)

(1) The translation product was mixed with Nano-Glo (registered trademark) Lusiferase Assay System (manufactured by Promega Corporation), and the luminescence intensity was measured by GloMax Explorer (manufactured by Promega Corporation).

### (Results)

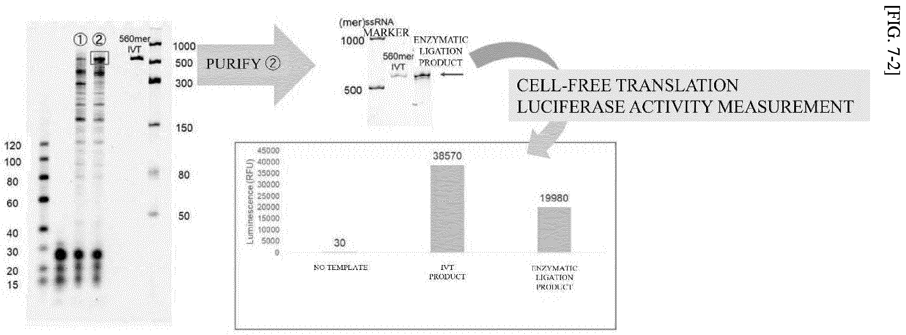

FIG. 7 shows Denatured-PAGE results and activity measurement results.

In both the tests of 1 and 2, a 560 mer full-length reaction product was confirmed. In the test of 2, the amount of full-length reaction product was confirmed to be large. After HPLC purification of the ligation reaction product of 2, the purified product was subjected to an in vitro translation reaction. It was found that when the translation solution was subjected to a luciferase assay and luminescence activity was measured, luminescence was observed, and the protein translated from the ligation reaction product had activity.

### (Example 7) Comparison of polynucleotide fragment molecule lengths and spacing of nucleic acid splints

In the present Example, experiments were conducted to compare the lengths of polynucleotide fragment molecules and examine the spacing of nucleic acid splints.

### (Materials and procedure)

### (Materials)

**[Table 16-1]**

| | | | |
|---|---|---|---|
| 1 | U3-1_1 | | GGGAAGACUAUACUUUCAGGGAUCA (SEQ ID NO:167) |
| 2 | U3-2_2 | p | UUUCUAUAGUGUGUUACUAGA (SEQ ID NO: 168) |
| 3 | U3-3_2 | p | GAAGUUUCUCUGAACGUGUAGAGC (SEQ ID NO:169) |
| 4 | U3-4_1 | p | ACCGAAAACCACGAGGAAGAG (SEQ ID NO:170) |
| 5 | U3-5_1 | p | AGGUAGCGUUUUCUCCUGAGCGUGA (SEQ ID NO:171) |
| 6 | 133-6_1 | p | AGCCGGCUUUCUGGCGUUG (SEQ ID NO:172) |
| 7 | U3-7_1 | p | CUUGGCUGCAACUGCCGUCAGCCAU (SEQ ID NO:173) |
| 8 | US-8_1 | p | UGAUGAUCGUUCUUCUCUC (SEQ ID NO:174) |
| 9 | U3-9_1 | p | CGUAUUGGGGAGUGAGAGGGAGAGA (SEQ ID NO:175) |
| 10 | U3-10_1 | p | ACGCGCTUCUCTAGUGGU (SEQ ID NO:176) |
| 11 | U3-1-2_1 | | GGGAAGACUA UACUUUCAGG GAUCAUUUCU AUAGUGUGUU ACUAGA (SEQ ID NO: 1 7 7) |
| 12 | U3-2-3_1 | p | UUUCU AUAGUGUGUU ACUAGAGAAG UUUCUCUGAA CGUGUAGAGC (SEQ ID NO: 1 7 8) |
| 13 | SpU3-1_1 | | tgaaagtatagtcttccc (SEQ ID NO:179) |
| 14 | SpU3-2_1 | | acttctctagtaacacactatagaaatgatccc (SEQ ID NO:180) |
| 15 | SpU3-3_1 | | acgttcagagaa (SEQ ID NO:181) |
| 16 | SpU3-4_2 | | tacctctcttcctcgtggttttcggtgctctac (SEQ ID NO:182) |
| 17 | SpU3-5_2 | | aggagaaaacgc (SEQ ID NO:183) |
| 18 | SpU3-6_1 | | ccaagcaacgccagaaagccggcttcacgctc (SEQ ID NO:184) |
| 19 | SpU3-7_1 | | acggcagttgcag (SEQ ID NO:185) |
| 20 | SpU3-8_1 | | aatacggagagaagaacgatcatcaatggctg (SEQ ID NO:186) |
| 21 | SpU3-9_1 | | ctctcactcccc (SEQ ID NO:187) |
| 22 | SpU3-10_1 | | accactcagaccgcgttctctcc (SEQ ID NO:188) |
| 23 | SpU3-1_4 | | gaaagtatagtcttcc (SEQ ID NO:189) |
| 24 | SpU3-2_4 | | cttctctagtaacacactatagaaatgatcc (SEQ ID NO:190) |
| 25 | SpU3-3_4 | | cgttcagaga (SEQ ID NO. 1 9 1) |
| 26 | SpU3-4_4 | | acctctcttcctcgtggttttcggtgctcta (SEQ ID NO:192) |
| 27 | SpU3-5_4 | | ggagaaaacg (SEQ ID NO:1 9 3) |
| 28 | SpU3-6_4 | | caagcaacgccagaaagccggcttcacgct (SEQ ID NO:194) |
| 29 | SpU3-7_4 | | cggcagttgca (SEQ ID NO:195) |
| 30 | SpU3-8_4 | | atacggagagaagaacgatcatcaatggct (SEQ ID NO:196) |
| 31 | SpU3-9 4 | | tetcactece (SEQ ID NO:197) |
| 32 | SpU3-10_4 | | ccactcagaccgcgttctctc (SEQ ID NO:198) |
| 33 | SpU3-1_18 | | ctatagaaatgatccctg (SEQ ID NO:199) |

**[Table 16-2]**

| | | | |
|---|---|---|---|
| 34 | SpU3-2_18 | | agaaacttctetagtaac (SEQ ID NO:200) |
| 35 | SpU3-3_18 | | gttttcggtgctctacac (SEQ ID NO:201) |
| 36 | SpU3-4_18 | | acgctacctctcttcctc (SEQ ID NO:202) |
| 37 | SpU3-5_18 | | aagccggcttcacgctca (SEQ ID NO:203) |
| 38 | SpU3-6_18 | | gcagccaagcaacgccag (SEQ ID NO:204) |
| 39 | SpU3-7_18 | | cgatcatcaatggctgac (SEQ ID NO:205) |
| 40 | SpU3-8_18 | | cccaatacggagagaaga (SEQ ID NO:206) |
| 41 | SpU3-9_18 | | agaccgcgttctctccct (SEQ ID NO:207) |
| 42 | SpU3-1_5 | | aaagtatagtcttccc (SEQ ID NO:208) |
| 43 | SpU3-2_5 | | actatagaaatgatccctg (SEQ ID NO:209) |
| 44 | SpU3-3_5 | | cagagaaacttctctagtaacac (SEQ ID NO:210) |
| 45 | SpU3-4_5 | | tggttttcggtgctctacacgtt (SEQ ID NO:211) |
| 46 | SpU3-5_5 | | gaaaacgctacctctcttcctcg (SEQ ID NO:212) |
| 47 | SpU3-6_5 | | aaagccggcttcacgctcagga (SEQ ID NO:213) |
| 48 | SpU3-7_5 | | gttgcagccaagcaacgccag (SEQ ID NO:214) |
| 49 | SpU3-8 5 | | cgatcatcaatggctgacggca (SEQ ID NO:215) |
| 50 | SpU3-9_5 | | actccccaatacggagagaagaa (SEQ ID NO:216) |
| 51 | SpU3-10_5 | | agaccgcgttctctccctctc (SEQ ID NO:217) |
| 52 | SpU3-11_5 | | accactc (SEQ ID NO:218) |
| 53 | SpU3-2-4_1 | | tacctctcttcctcgtggttttcggtgctctacacgttcagagaa acttctctagtaacacactatagaaatgatccc （SEQ ID NO: ２１９） |

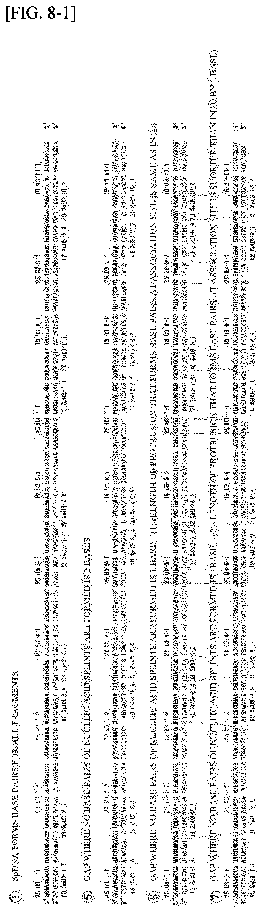

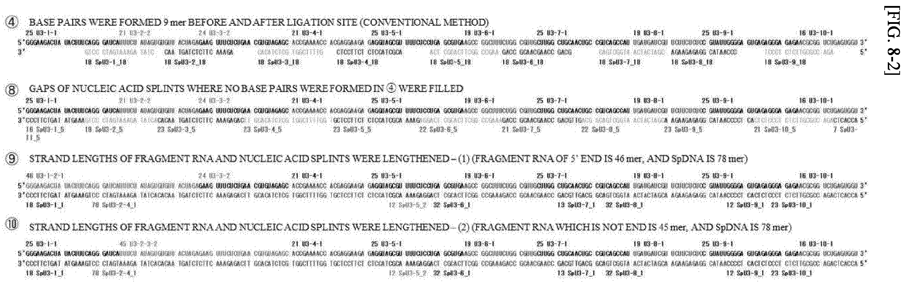

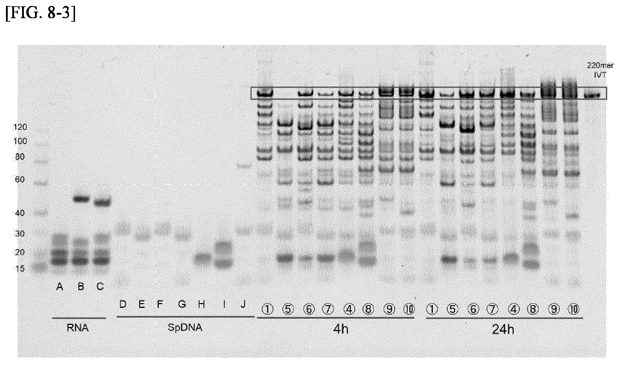

The sequences used are shown in FIG. 8-1 and FIG. 8-2.

### (Procedure)

Except for the used nucleic acid fragments shown in the above Table 16, the same materials as in Example 1 were used.

The combinations of nucleic acid fragments used are shown in Table 17.

**[Table 17]**

| | RNA | Nucleic acid splint |
|---|---|---|
| ① | 1~10 | 13~22 |
| ⑤ | 1~10 | 23~32 |
| ⑥ | 1~10 | 14, 16, 18, 20, 22, 23, 25, 27, 29, 31 |
| ⑦ | 1~10 | 13, 15, 17, 19, 21, 24, 26, 28, 30, 32 |
| ④ | 1~10 | 33~41 |
| ⑧ | 1~10 | 42~52 |
| ⑨ | 2~10,11 | 13, 53, 17~22 |
| ⑩ | 1,3~10,12 | 13, 53, 17~22 |

### (Results)

FIG. 6-2 shows the results of Denatured-PAGE. An enzymatic ligation product that seems to be identical to the 220 mer transcript was confirmed.

### (Example 8: Ligation of transcripts and chemically synthesized RNA using DNA splints)

Next, ligation of transcripts and chemically synthesized RNA using DNA splints was performed.

In the present Example, a ligation reaction was performed using a combination of transcription-synthesized long RNA and chemically synthesized RNA, and an attempt was made to apply the ligation reaction to synthesis of RNA of a longer strand. In addition, how the ligation efficiency was changed by assembling all the DNA splints outside the joining point was examined, and the superiority of this method was demonstrated.

### (Procedure)

### (Chemical synthesis)

(1) Oligo-nucleic acids other than NL537-p were synthesized by a usual phosphoramidite method.

### (Transcriptional synthesis of 537 mer RNA)

(1) A template ds DNA for transcriptional synthesis in which a T7 promoter sequence was added to the 5' end side was synthesized by a PCR method.
(2) 537 mer RNA was synthesized by in vitro transcription using the template synthesized in (1).
(3) The RNA synthesized in (2) was treated with RppH Pyrophosphatase (manufactured by New England Biolabs Inc.) to convert the 5' end of the RNA into a monophosphorylated.

### (Ligation reaction)

(1) The RNA strands and the nucleic acid splints were mixed, then heated at 80°C for 3 minutes, and then cooled to 5°C over 25 minutes.
(2) The nucleic acid strands and the reaction reagents were mixed at the following concentrations.

(1) The RNA strands and the nucleic acid splints were mixed, then heated at 80°C for 3 minutes, and then cooled to 5°C over 25 minutes.
(2) The nucleic acid strands and the reaction reagents were mixed at the following concentrations.
   1 (560 mer in total)
   1 µM RNA nucleic acid strands 37 kinds (see the table below)
   1.5 µM nucleic acid splints 37 kinds (see the table below)
   50 mM Tris-HCl (pH7.5)
   2 mM MgCl₂
   0.4 mM ATP
   1 mM DTT
   5% PEG8000
(3) The solution mixed in (2) was subjected to a ligation reaction at 25°C.

### (Denatured-PAGE)

Details of the procedure are as follows.
(1) 1 µL of the enzymatic ligation reaction sample was mixed with bromophenol blue-containing formamide and heated at 80°C for 5 minutes. The heated sample was quenched immediately in ice.
(2) 2 µL of the solution of (1) was applied to a 5% acrylamide gel containing 7.5M urea, 1xTBE, and electrophoresed using 1xTBE buffer at a voltage of 300 V for 15 minutes or 70 minutes.
(3) The gel after electrophoresis was stained with SYBR Gold (manufactured by Thermo Fisher Scientific, Inc.), and then photographed with a transilluminator.
(4) The band intensity of the ligation product in the captured electrophoresis image was measured, and the relative intensity was quantified.

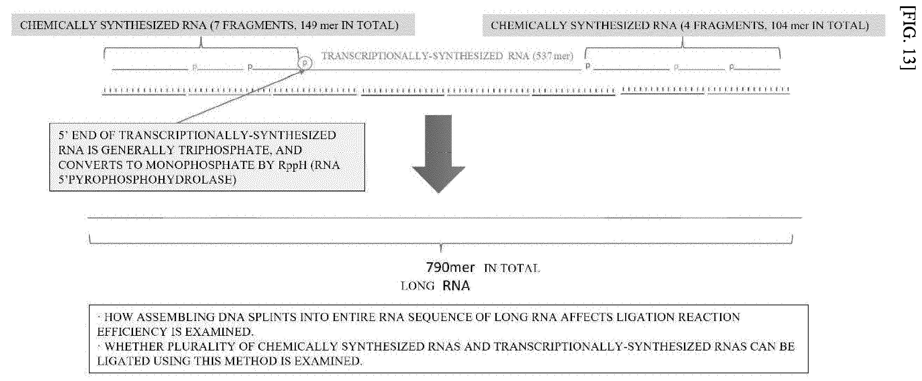

Details of the procedure are shown in FIG. 13.

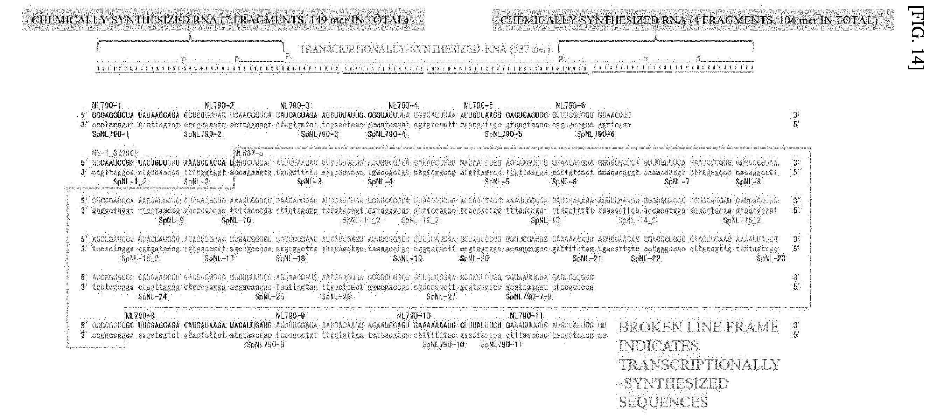

A schematic diagram of the target product and the starting substance is shown in FIG. 14.

### (Ligation reaction test of transcripts and chemically synthesized RNA using DNA splints)

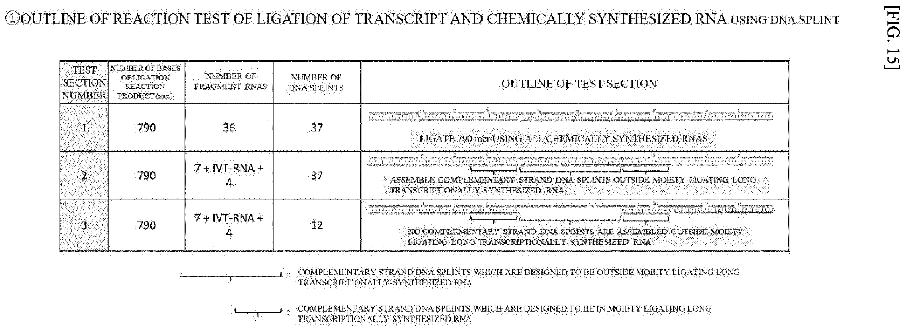

Next, an outline of a ligation reaction test of transcripts and chemically synthesized RNA using DNA splints is shown (FIG. 15).

The sequence information used is shown below.

**[Table 18]**

| | | | | |
|---|---|---|---|---|
| 1 | NL790-1 | | GGGAGGUCUAUAUAAGCAGAGCUCG | SEQ ID NO:220 |
| 2 | NL790-2 | p | UUUAGUGAACCGUCAG | SEQ ID NO: 22 1 |
| 3 | NL790-3 | p | AUCACUAGAAGCUUUAUUGCGGUA | SEQ ID NO:222 |
| 4 | NL790-4 | p | GUUUAUCACAGUUAAA | SEQ ID NO:223 |
| 5 | NL790-5 | p | UUGCUAACGCAGUCAGUGGG | SEQ ID NO:224 |
| 6 | NL790-6 | p | CCUCGGCGGCCAAGCUU | SEQ ID NO:225 |
| 7 | NL790-8 | p | GCUUCGAGCAGACAUGAUAAGAUACAUUGAUG | SEQ ID NO:226 |
| 8 | NL790-9 | p | AGUUUGGACAAACCACAACUAGAAUGC | SEQ ID No:227 |
| 9 | NL790-10 | p | AGUGAAAAAAAUGCUUUAUUUGUG | SEQ ID NO:228 |
| 10 | NL790-11 | p | AAAUUUGUGAUGCUAUUGCUU | SEQ ID NO:229 |
| 11 | NL-1_3(790) | p | GGCAAUCCGGUACUGUUGGUAAAGCCACCAU | SEQ ID NO: 230 |
| 12 | NL-2-3_3(790) | p | GGUCUUCACACUCGAAGAUUUCGUUGGGGACUGG | SEQ ID NO: 243 |

**[Table 19]**

| | | | | |
|---|---|---|---|---|
| 13 | NL537 ⁻p | p | GGUCUUCACACUCGAAGAUUUCGUUGGGGACUGGCG ACAGACAGCCGGCUACAACCUGGACCAAGUCCUUGAA CAGGGAGGUGUGUCCAGUUUGUUUCAGAAUCUCGGG GUGUCCGUAACUCCGAUCCAAAGGAUUGUCCUGAGC GGUGAAAAUGGGCUGAAGAUCGACAUCCAUGUCAUCA UCCCGUAUGAAGGUCUGAGCGGCGACCAAAUGGGCC AGAUCGAAAAAAUUUUUAAGGUGGUGUACCCUGUGGA UGAUCAUCACUUUAAGGUGAUCCUGCACUAUGGCACA CUGGUAAUCGACGGGGUUACGCCGAACAUGAUCGAC UAUUUCGGACGGCCGUAUGAAGGCAUCGCCGUGUUC GACGGCAAAAAGAUCACUGUAACAGGGACCCUGUGGA ACGGCAACAAAAUUAUCGACGAGCGCCUGAUCAACCC CGACGGCUCCCUGCUGUUCCGAGUAACCAUCAACGG AGUGACCGGCUGGCGGCUGUGCGAACGCAUUCUGGC GUAAUUCUAGAGUCGGGGCGGCCGGCC | SEQ ID NO: 231 |

**[Table 20]**

| | | | | |
|---|---|---|---|---|
| 14 | SpNL790-1 | | tctgcttatatagacctccc | SEQ ID NO: 232 |
| 15 | SpNL790-2 | | gtgatctgacggttcactaaacgagc | SEQ ID NO: 233 |
| 16 | SpNL790-3 | | caataaagcttcta | SEQ ID NO: 234 |
| 17 | SpNL790-4 | | agcaatttaactgtgataaactacce | SEQ ID NO: 235 |
| 18 | SpNL790-5 | | cgaggcccactgactgcgtt | SEQ ID NO: 236 |
| 19 | SpNL790-6 | | ttgccaagcttggccgc | SEQ ID NO:237 |
| 20 | SpNL790-9 | | tcactgcattctagttgtggtttgtccaaactcatcaa | SEQ ID NO: 238 |
| 21 | SpNL790-10 | | taaagcatttttt | SEQ ID NO: 239 |
| 22 | SpNL790-11 | | aagcaatagcatcacaaatttcacaaa | SEQ ID NO: 240 |
| 23 | SpNL-1_2 | | accaacagtaccgga | SEQ ID NO: 241 |
| 24 | SpNL790-7-8 | | tgtatcttatcatgtctgctcgaagcggccggccgccccgactctagaattacg | SEQ ID NO: 242 |

**[Table 21]**

| | | | | |
|---|---|---|---|---|
| 25 | SpNL-3_1 | | ccccaacgaaatctt | SEQ ID NO:141 |
| 26 | SpNL-4_1 | | gttgtagccggctgtctgtcgccagt | SEQ ID NO: 142 |
| 27 | SpNL-5_1 | | aggacttggtccag | SEQ ID NO: 143 |
| 28 | SpNL-6_1 | | caaactggacacacctccctgttca | SEQ ID NO: 144 |
| 29 | SpNL-7_1 | | cccgagattctgaaa | SEQ ID NO: 145 |
| 30 | SpNL-8_1 | | tcctttggatcggagttacggacac | SEQ ID NO: 146 |
| 31 | SpNL-9_1 | | accgctcaggacaa | SEQ ID NO:147 |
| 32 | SpNL-10_1 | | tggatgtcgatcttcagcccattttc | SEQ ID NO:148 |
| 33 | SpNL-11_2 | | cgggatgatgaca | SEQ ID NO:149 |
| 34 | SpNL-12_2 | | ccatttggtcgccgctcagaccttcata | SEQ ID NO:150 |
| 35 | SpNL-13_1 | | aaaattttttcgatctggc | SEQ ID NO: 151 |
| 36 | SpNL-14_2 | | catccacagggtacaccacctta | SEQ ID NO:152 |
| 37 | SpNL-15_2 | | atcaccttaaagtgatgat | SEQ ID NO:153 |
| 38 | SpNL-16_2 | | agtgtgccatagtgcagg | SEQ ID NO: 154 |
| 39 | SpNL-17_1 | | accccgtcgattacc | SEQ ID NO:155 |
| 40 | SpNL-18_1 | | cgaaatagtcgatcatgttcggcgta | SEQ ID NO: 156 |
| 41 | SpNL-19_1 | | ttcatacggccgtc | SEQ ID NO:157 |
| 42 | SpNL-20_1 | | ttttgccgtcgaacacggcgatgcc | SEQ ID NO: 158 |
| 43 | SpNL-21_1 | | gttacagtgatct | SEQ ID NO:159 |
| 44 | SpNL-22_1 | | attttgttgccgttccacagggtccct | SEQ ID NO:160 |
| 45 | SpNL-23_1 | | aggcgctcgtcgata | SEQ ID NO:161 |
| 46 | SpNL-24_1 | | acagcagggagccgtcggggttgatc | SEQ ID NO: 162 |
| 47 | SpNL-25_1 | | gatggttactcgga | SEQ ID NO: 163 |
| 48 | SpNL-26_1 | | agccgccagccggtcactccgtt | SEQ ID NO:164 |
| 49 | SpNL-27_1 | | ccagaatgcgttcgcac | SEQ ID NO:165 |

### (Results)

The results are shown in FIG. 16.

In the present Example, as shown in FIG. 16, 36 fragments and 790 mer in total were ligated. A product longer than that of the above Example (560 mer) could be synthesized using a technique other than the RNA synthesis method by ligation of transcriptionally-synthesized RNA and chemically synthesized RNA. (1) As shown in FIG. 16, it was found that the ligation reaction proceeds when the DNA splints are also assembled outside the joining point. (2) In addition, the ligation reaction is less likely to proceed unless a complementary strand is assembled outside the joining point. Non-specific ligations were also found to occur (3).

In this way, the superiority of the method of the present disclosure was shown, in which DNA splints were assembled to the entire RNA sequence.

From the results of the present Example, without intending to be limited, it is understood that the ligation reaction is likely to proceed when the DNA splints are assembled with the long RNA at all sites other than the joining point. In addition, although not intended to be limited, it is understood to be not preferable that complementary strands are not assembled outside the joining point because the ligation reaction hardly proceeds and non-specific ligation also occurs. Although not intended to be limited, it has been shown that it is effective to assemble complementary strands at all moieties of long RNA using short DNA splint even in a ligation reaction of a transcript having a long length.

### (Ligation of transcripts and chemically synthesized RNA using DNA splints)

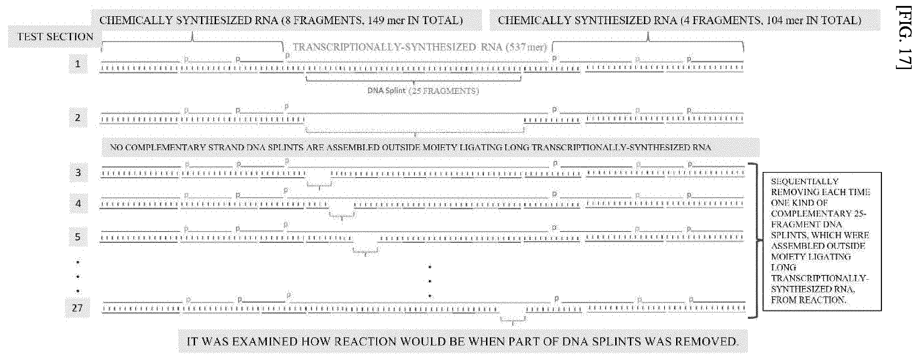

As shown in FIG. 17, an experiment of ligation of transcripts and chemically synthesized RNA using DNA splints was performed. Here, it was examined how the reaction would be when a part of the DNA splints was removed. It is generally as follows.

As shown in the test sections 1 to 27 of FIG. 17, experiments were conducted by sequentially removing one kind of complementary 25-fragment DNA splints, which were assembled outside the moiety ligating the long transcriptionally-synthesized RNA, from the reaction. That is, the procedure was performed in a manner that complementary DNA splints were not assembled outside the moiety ligating the long transcriptionally-synthesized RNA.

The DNA splint combinations are as follows.

### (Test section / used nucleic acid sequence number)

**[Table 22]**

| Test section number | RNA | DNA Splint |
|---|---|---|
| 1 | 1~11, 13 | 14~49 |
| 2 | 1~11, 13 | 14~24 |
| 3 | 1~11, 13 | 14~24, 26~49 |
| 4 | 1-11, 13 | 14~25, 27~49 |
| 5 | 1~11, 13 | 14~26, 28-49 |
| 6 | 1~11, 13 | 14~27, 29~49 |
| 7 | 1~11, 13 | 14~28, 30~49 |
| 8 | 1~11, 13 | 14~29, 31~49 |
| 9 | 1~11, 13 | 14~30, 32~49 |
| 10 | 1~11, 13 | 14~31, 33~49 |
| 11 | 1~11, 13 | 14~32, 34~49 |
| 12 | 1~11, 13 | 14~33, 35~49 |
| 13 | 1~11, 13 | 14~34, 36~49 |
| 14 | 1~11, 13 | 14~35, 37~49 |
| 15 | 1~11, 13 | 14~36, 38~49 |
| 16 | 1~11, 13 | 14~37, 39~49 |
| 17 | 1~11, 13 | 14~38, 40~49 |
| 18 | 1~11, 13 | 14~39, 41~49 |
| 19 | 1~11, 13 | 14~40, 42~49 |
| 20 | 1~11, 13 | 14~41, 43~49 |
| 21 | 1~11, 13 | 14~42, 44~49 |
| 22 | 1~11, 13 | 14~43, 45~49 |
| 23 | 1~11, 13 | 14~44, 46~49 |
| 24 | 1~11, 13 | 14~45, 47~49 |
| 25 | 1~11, 13 | 14~46, 48~49 |
| 26 | 1~11, 13 | 14~47, 49 |
| 27 | 1~11, 13 | 14~48 |

### (Results)

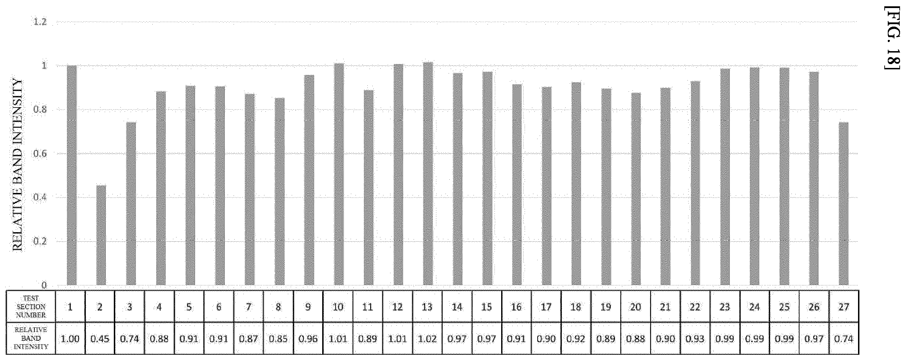

The results are shown in FIG. 18. FIG. 18 shows Denatured-PAGE results and relative band intensities. As illustrated, it was shown that, as a result of conducting experiments by sequentially removing each time one kind of complementary 25-fragment DNA splints, which were assembled outside the moiety ligating the long transcriptionally-synthesized RNA, from the reaction, when there was a moiety where DNA splints were not assembled, the reaction efficiency changed depending on the position of the moiety.

In addition, when there was a moiety where DNA splints were not assembled in the vicinity of the ligation site, a decrease in reaction efficiency was observed. This decrease in reaction efficiency is considered to be within an acceptable range, but considering the time and cost for examining the DNA splints at which positions can be removed, it is considered that efficiency is better when the DNA splints were assembled outside the moiety ligating the long transcriptionally-synthesized RNA.

### (Example 9: Formulation example)

The compounds of the present disclosure may be provided in appropriate dosage forms. Examples thereof will be described below.

Examples include tablets, capsules, powders, granules, solutions, suspensions, injections, patches, cataplasms, and the like. In addition, these formulations can be produced by a known method, such as appropriately mixing these using additives used as normal pharmaceutical additives.

### (Example 10: Animal experiment)

Mice with disease (for example, tumor-bearing mice) are administered the polynucleotide of the present disclosure or a placebo intraperitoneally.

Then, the placebo can be compared to the polynucleotide to assess the efficacy of the polynucleotide of the present disclosure against the disease in the mouse body.

### (Note)

As described above, although the present disclosure has been illustrated using the preferred embodiments of the present disclosure, it is understood that the scope of the present disclosure should be interpreted only by the claims. It is understood that the patents, patent applications, and other documents cited in the present description are to be incorporated by reference in the present description as if their contents were specifically described in the present description in detail. The present application claims priority to Japanese Patent Application No. 2022-138632 filed on August 31, 2022 to the Japan Patent Office, the entire contents of which are incorporated in the present application by reference as necessary.

### Industrial Applicability

The technique provided in the present disclosure can be utilized in any field utilizing a technique of ligating polynucleotides.

### [Sequence listing free text]

SEQ ID NO: 1: sg1
SEQ ID NO: 2: sg2
SEQ ID NO: 3: sg3
SEQ ID NO: 4: sg4
SEQ ID NO: 5: sg5
SEQ ID NO: 6: sg6
SEQ ID NO: 7: Spsg1
SEQ ID NO: 8: Spsg2
SEQ ID NO: 9: Spsg3
SEQ ID NO: 10: Spsg4
SEQ ID NO: 11: Spsg1_16
SEQ ID NO: 12: Spsg2_14
SEQ ID NO: 13: Spsg3_18
SEQ ID NO: 14: Spsg4_14
SEQ ID NO: 15: Spsg5_14
SEQ ID NO: 16: Spsg100
SEQ ID NO: 17: sg1_1
SEQ ID NO: 18: sg2_1
SEQ ID NO: 19: sg3_1
SEQ ID NO: 20: sg4_1
SEQ ID NO: 21: sg5_1
SEQ ID NO: 22: sg6_1
SEQ ID NO: 23: sg1_1_PS_OMe_3
SEQ ID NO: 24: sg6_1_PS_OMe_3
SEQ ID NO: 25: sg1_1_C20
SEQ ID NO: 26: sg2_1_C20
SEQ ID NO: 27: sg3_1_T2
SEQ ID NO: 28: sg4_1_T2
SEQ ID NO: 29: sg5_1_T2
SEQ ID NO: 30: sg6_1_T2
SEQ ID NO: 31: sg3_1_T8
SEQ ID NO: 32: sg4_1_T8
SEQ ID NO: 33: sg2_1_C20_LNA
SEQ ID NO: 34: sg2_1_C20_BNA
SEQ ID NO: 35: sg2_1_C20_MOE
SEQ ID NO: 36: Spsg1_2
SEQ ID NO: 37: Spsg2_2
SEQ ID NO: 38: Spsg_3_1
SEQ ID NO: 39: Spsg_4_1
SEQ ID NO: 40: Natural form
SEQ ID NO: 41: Terminal PS, 2'-OMe conversion
SEQ ID NO: 42: Highly modified 1.
SEQ ID NO: 43: Highly modified 2.
SEQ ID NO: 44: Highly modified 3. Containing LNA
SEQ ID NO: 45: Highly modified 4. Containing BNA-NC (N-Me)
SEQ ID NO: 46: Highly modified 5. Containing 2'-MOE
SEQ ID NO: 47: U3-1_1
SEQ ID NO: 48: U3-2_2
SEQ ID NO: 49: U3-3_2
SEQ ID NO: 50: U3-4_1
SEQ ID NO: 51: U3-5_1
SEQ ID NO: 52: U3-6_1
SEQ ID NO: 53: U3-7_1
SEQ ID NO: 54: U3-8_1
SEQ ID NO: 55: U3-9_1
SEQ ID NO: 56: U3-10_1
SEQ ID NO: 57: SpU3-1_1
SEQ ID NO: 58: SpU3-2_1
SEQ ID NO: 59: SpU3-3_1
SEQ ID NO: 60: SpU3-4_2
SEQ ID NO: 61: SpU3-5_2
SEQ ID NO: 62: SpU3-6_1
SEQ ID NO: 63: SpU3-7_1
SEQ ID NO: 64: SpU3-8_1
SEQ ID NO: 65: SpU3-9_1
SEQ ID NO: 66: SpU3-10_1
SEQ ID NO: 67: SpU3-1_1
SEQ ID NO: 68: SpU3-2_1
SEQ ID NO: 69: SpU3-3_1
SEQ ID NO: 70: SpU3-4_2
SEQ ID NO: 71: SpU3-5_2
SEQ ID NO: 72: SpU3-6_1
SEQ ID NO: 73: SpU3-7_1
SEQ ID NO: 74: SpU3-8_1
SEQ ID NO: 75: SpU3-9_1
SEQ ID NO: 76: SpU3-10_1
SEQ ID NO: 77: SpU3-1_13
SEQ ID NO: 78: SpU3-2_1
SEQ ID NO: 79: SpU3-3_1
SEQ ID NO: 80: SpU3-4_2
SEQ ID NO: 81: SpU3-5_2
SEQ ID NO: 82: SpU3-6_1
SEQ ID NO: 83: SpU3-7_1
SEQ ID NO: 84: SpU3-8_1
SEQ ID NO: 85: SpU3-9_1
SEQ ID NO: 86: SpU3-10_18
SEQ ID NO: 87: SpU3-2_1
SEQ ID NO: 88: SpU3-4_2
SEQ ID NO: 89: SpU3-6_1
SEQ ID NO: 90: SpU3-8_1
SEQ ID NO: 91: SpU3-10_1
SEQ ID NO: 92: SpU3-1_18
SEQ ID NO: 93: SpU3-2_18
SEQ ID NO: 94: SpU3-3_18
SEQ ID NO: 95: SpU3-4_18
SEQ ID NO: 96: SpU3-5_18
SEQ ID NO: 97: SpU3-6_18
SEQ ID NO: 98: SpU3-7_18
SEQ ID NO: 99: SpU3-8_18
SEQ ID NO: 100: SpU3-9_18
SEQ ID NO: 101: SpU3-1_4
SEQ ID NO: 102: SpU3-2_4
SEQ ID NO: 103: SpU3-3_4
SEQ ID NO: 104: SpU3-4_4
SEQ ID NO: 105: SpU3-5_4
SEQ ID NO: 106: SpU3-6_4
SEQ ID NO: 107: SpU3-7_4
SEQ ID NO: 108: SpU3-8_4
SEQ ID NO: 109: SpU3-9_4
SEQ ID NO: 110: SpU3-10_4
SEQ ID NO: 111: NL-1_1
SEQ ID NO: 112: NL-2_1
SEQ ID NO: 113: NL-3_1
SEQ ID NO: 114: NL-4_1
SEQ ID NO: 115: NL-5_2
SEQ ID NO: 116: NL-6_2
SEQ ID NO: 117: NL-7_1
SEQ ID NO: 118: NL-8_1
SEQ ID NO: 119: NL-9_1
SEQ ID NO: 120: NL-10_1
SEQ ID NO: 121: NL-11_4
SEQ ID NO: 122: NL-12_4
SEQ ID NO: 123: NL-13_2
SEQ ID NO: 124: NL-14_2
SEQ ID NO: 125: NL-15_2
SEQ ID NO: 126: NL-16_2
SEQ ID NO: 127: NL-17_1
SEQ ID NO: 128: NL-18_1
SEQ ID NO: 129: NL-19_1
SEQ ID NO: 130: NL-20_1
SEQ ID NO: 131: NL-21_1
SEQ ID NO: 132: NL-22_1
SEQ ID NO: 133: NL-23_1
SEQ ID NO: 134: NL-24_1
SEQ ID NO: 135: NL-25_1
SEQ ID NO: 136: NL-26_1
SEQ ID NO: 137: NL-27_1
SEQ ID NO: 138: NL-28_1
SEQ ID NO: 139: SpNL-1_1
SEQ ID NO: 140: SpNL-2_1
SEQ ID NO: 141: SpNL-3_1
SEQ ID NO: 142: SpNL-4_1
SEQ ID NO: 143: SpNL-5_1
SEQ ID NO: 144: SpNL-6_1
SEQ ID NO: 145: SpNL-7_1
SEQ ID NO: 146: SpNL-8_1
SEQ ID NO: 147: SpNL-9_1
SEQ ID NO: 148: SpNL-10_1
SEQ ID NO: 149: SpNL-11_2
SEQ ID NO: 150: SpNL-12_2
SEQ ID NO: 151: SpNL-13_1
SEQ ID NO: 152: SpNL-14_2
SEQ ID NO: 153: SpNL-15_2
SEQ ID NO: 154: SpNL-16_2
SEQ ID NO: 155: SpNL-17_1
SEQ ID NO: 156: SpNL-18_1
SEQ ID NO: 157: SpNL-19_1
SEQ ID NO: 158: SpNL-20_1
SEQ ID NO: 159: SpNL-21_1
SEQ ID NO: 160: SpNL-22_1
SEQ ID NO: 161: SpNL-23_1
SEQ ID NO: 162: SpNL-24_1
SEQ ID NO: 163: SpNL-25_1
SEQ ID NO: 164: SpNL-26_1
SEQ ID NO: 165: SpNL-27_1
SEQ ID NO: 166: SpNL-28_1
SEQ ID NO: 167: U3-1_1
SEQ ID NO: 168: U3-2_2
SEQ ID NO: 169: U3-3_2
SEQ ID NO: 170: U3-4_1
SEQ ID NO: 171: U3-5_1
SEQ ID NO: 172: U3-6_1
SEQ ID NO: 173: U3-7_1
SEQ ID NO: 174: U3-8_1
SEQ ID NO: 175: U3-9_1
SEQ ID NO: 176: U3-10_1
SEQ ID NO: 177: U3-1-2_1
SEQ ID NO: 178: U3-2-3_1
SEQ ID NO: 179: SpU3-1_1
SEQ ID NO: 180: SpU3-2_1
SEQ ID NO: 181: SpU3-3_1
SEQ ID NO: 182: SpU3-4_2
SEQ ID NO: 183: SpU3-5_2
SEQ ID NO: 184: SpU3-6_1
SEQ ID NO: 185: SpU3-7_1
SEQ ID NO: 186: SpU3-8_1
SEQ ID NO: 187: SpU3-9_1
SEQ ID NO: 188: SpU3-10_1
SEQ ID NO: 189: SpU3-1_4
SEQ ID NO: 190: SpU3-2_4
SEQ ID NO: 191: SpU3-3_4
SEQ ID NO: 192: SpU3-4_4
SEQ ID NO: 193: SpU3-5_4
SEQ ID NO: 194: SpU3-6_4
SEQ ID NO: 195: SpU3-7_4
SEQ ID NO: 196: SpU3-8_4
SEQ ID NO: 197: SpU3-9_4
SEQ ID NO: 198: SpU3-10_4
SEQ ID NO: 199: SpU3-1_18
SEQ ID NO: 200: SpU3-2_18
SEQ ID NO: 201: SpU3-3_18
SEQ ID NO: 202: SpU3-4_18
SEQ ID NO: 203: SpU3-5_18
SEQ ID NO: 204: SpU3-6_18
SEQ ID NO: 205: SpU3-7_18
SEQ ID NO: 206: SpU3-8_18
SEQ ID NO: 207: SpU3-9_18
SEQ ID NO: 208: SpU3-1_5
SEQ ID NO: 209: SpU3-2_5
SEQ ID NO: 210: SpU3-3_5
SEQ ID NO: 211: SpU3-4_5
SEQ ID NO: 212: SpU3-5_5
SEQ ID NO: 213: SpU3-6_5
SEQ ID NO: 214: SpU3-7_5
SEQ ID NO: 215: SpU3-8_5
SEQ ID NO: 216: SpU3-9_5
SEQ ID NO: 217: SpU3-10_5
SEQ ID NO: 218: SpU3-11_5
SEQ ID NO: 219: SpU3-2-4_1
SEQ ID NO: 220: NL790-1
SEQ ID NO: 221: NL790-2
SEQ ID NO: 222: NL790-3
SEQ ID NO: 223: NL790-4
SEQ ID NO: 224: NL790-5
SEQ ID NO: 225: NL790-6
SEQ ID NO: 226: NL790-8
SEQ ID NO: 227: NL790-9
SEQ ID NO: 228: NL790-10
SEQ ID NO: 229: NL790-11
SEQ ID NO: 230: NL-1_3(790)
SEQ ID NO: 231: NL537-p
SEQ ID NO: 232: SpNL790-1
SEQ ID NO: 233: SpNL790-2
SEQ ID NO: 234: SpNL790-3
SEQ ID NO: 235: SpNL790-4
SEQ ID NO: 236: SpNL790-5
SEQ ID NO: 237: SpNL790-6
SEQ ID NO: 238: SpNL790-9
SEQ ID NO: 239: SpNL790-10
SEQ ID NO: 240: SpNL790-11
SEQ ID NO: 241: SpNL-1_2
SEQ ID NO: 242: SpNL790-7-8
SEQ ID NO: 243: NL-2-3_3(790)

### [Sequence listing]

International Application No. 093396 under the Patent Cooperation Treaty: Method for Producing Nucleic Acid MoleculeJP23031631 20230830-00481040552301861332 Normal 20230830163754202308171402394600_P1AP101_09_25.xml

## Claims

1. A method for producing a polynucleotide molecule having a specific sequence, the method comprising:
1) providing two or more polynucleotide fragment molecules including a portion of the specific sequence; and
2) ligating the two or more polynucleotide fragment molecules in the presence of two or more nucleic acid splints, wherein
each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

2. The method according to claim 1, wherein there are three or more of the polynucleotide fragment molecules used.

3. The method according to claim 1, wherein the nucleic acid splints are 8 mer or more complementary to the polynucleotide molecule.

4. The method according to claim 1, wherein a distance between a nucleic acid splint aligned with the specific sequence and a nucleic acid splint adjacent to the nucleic acid splint aligned with the specific sequence is 2 bases or less.

5. The method according to claim 1, wherein the deletions are 2 bases or less.

6. The method according to claim 1, wherein the nucleic acid splint has a sequence that protrudes from a joining point of a polynucleotide fragment molecule.

7. The method according to claim 1, wherein at least one end of the polynucleotide fragment molecule has an association with a carrier.

8. The method according to claim 7, wherein the carrier is polystyrene, CPG (Controlled pore glass), or magnetic beads.

9. A polynucleotide molecule produced by the method according to any one of claims 1 to 8.

10. A kit for producing a polynucleotide molecule having a specific sequence, the kit comprising:
1) two or more polynucleotide fragment molecules including a portion of the specific sequence; and
2) two or more nucleic acid splints, wherein
each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

11. The kit according to claim 10, wherein the nucleic acid splints are 8 mer or more complementary to the polynucleotide molecule.

12. The kit according to claim 10, wherein a distance between a nucleic acid splint aligned with the specific sequence and an adjacent nucleic acid splint aligned with the specific sequence is 2 bases or less.

13. The kit according to claim 10, wherein the deletions are 2 bases or less.

14. The method according to claim 10, wherein the nucleic acid splint has a sequence that protrudes from a joining point of a polynucleotide fragment molecule.

15. The kit according to claim 10, wherein at least one end of the polynucleotide fragment molecule has an association with a carrier.

16. The kit according to claim 15, wherein the carrier is polystyrene, CPG (Controlled pore glass), or magnetic beads.

17. A method for producing a polynucleotide molecule having a specific sequence, the method comprising:
A) designing two or more polynucleotide fragment molecules including a portion of the desired sequence based on the specific sequence;
B) designing two or more nucleic acid splints based on the specific sequence;
C) providing the polynucleotide fragment molecules and the nucleic acid splints which have been designed; and
D) ligating the polynucleotide fragment molecules which have been designed in the presence of the nucleic acid splints which have been designed, wherein
each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

18. A method for producing a polypeptide fragment and a nucleic acid splint for producing a polynucleotide molecule having a specific sequence, the method comprising:
A) designing two or more polynucleotide fragment molecules including a portion of the desired sequence based on the specific sequence;
B) designing two or more nucleic acid splints based on the specific sequence; and
C) producing the polynucleotide fragment molecules and the two or more nucleic acid splints which have been designed, wherein
each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

19. The method according to claim 17 or claim 18, wherein the nucleic acid splints are 8 mer or more complementary to the polynucleotide molecule.

20. The method according to claim 17 or claim 18, wherein a distance between a nucleic acid splint aligned with the specific sequence and a nucleic acid splint adjacent to the nucleic acid splint aligned with the specific sequence is 2 bases or less.

21. The method according to claim 17 or claim 18, wherein the deletions are 2 bases or less.

22. The method according to claim 17 or claim 18, wherein the nucleic acid splint has a sequence that protrudes from a joining point of a polynucleotide fragment molecule.

23. The method according to claim 17, wherein at least one end of the polynucleotide fragment molecule has an association with a carrier.

24. The method according to claim 23, wherein the carrier is polystyrene, CPG (Controlled pore glass), or magnetic beads.

25. A method of designing a polypeptide fragment and a nucleic acid splint for producing a polynucleotide molecule having a specific sequence, the method comprising:
A) designing two or more polynucleotide fragment molecules including a portion of the desired sequence based on the specific sequence; and
B) designing two or more nucleic acid splints based on the specific sequence,
wherein
each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

26. The method according to claim 25, wherein the nucleic acid splints are 8 mer or more complementary to the polynucleotide molecule.

27. The method according to claim 25, wherein a distance between a nucleic acid splint aligned with the specific sequence and a nucleic acid splint adjacent to the nucleic acid splint aligned with the specific sequence is 2 bases or less.

28. The method according to claim 27, wherein the deletions are 2 bases or less.

29. The method according to claim 25, wherein the nucleic acid splints are 10 mer to 50 mer.

30. The method according to claim 25, wherein the polynucleotide fragment molecules are 10 mer to 50 mer.

31. The method according to claim 25, wherein the nucleic acid splint has a sequence that protrudes from a joining point of the polynucleotide fragment molecule.

32. The method according to claim 25, wherein at least one end of the polynucleotide fragment molecule has an association with a carrier.

33. The method according to claim 32, wherein the carrier is polystyrene, CPG (Controlled pore glass), or magnetic beads.

34. A program for causing a computer to execute a method of designing a polypeptide fragment and a nucleic acid splint for producing a polynucleotide molecule having a specific sequence, the method including:
A) designing two or more polynucleotide fragment molecules including a portion of the desired sequence based on the specific sequence; and
B) designing two or more nucleic acid splints based on the specific sequence,
wherein
each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

35. The program according to claim 34, wherein the nucleic acid splints are 8 mer or more complementary to the polynucleotide molecule.

36. The program according to claim 34, wherein a distance between a nucleic acid splint aligned with the specific sequence and a nucleic acid splint adjacent to the nucleic acid splint aligned with the specific sequence is 2 bases or less.

37. The program according to claim 34, wherein the deletions are 2 bases or less.

38. The program according to claim 34, wherein the nucleic acid splints are 10 mer to 50 mer.

39. The program according to claim 34, wherein the polynucleotide fragment molecules are 10 mer to 50 mer.

40. The program according to claim 34, wherein the nucleic acid splint has a sequence that protrudes from a joining point of the polynucleotide fragment molecule.

41. The program according to claim 34, wherein at least one end of the polynucleotide fragment molecule has an association with a carrier.

42. The program according to claim 41, wherein the carrier is polystyrene, CPG (Controlled pore glass), or magnetic beads.

43. A recording medium storing a program for causing a computer to execute a method of designing a polypeptide fragment and a nucleic acid splint for producing a polynucleotide molecule having a specific sequence, the method including:
A) designing two or more polynucleotide fragment molecules including a portion of the desired sequence based on the specific sequence; and
B) designing two or more nucleic acid splints based on the specific sequence,
wherein
each sequence of the two or more polynucleotide fragment molecules when assembled constitutes the specific sequence,
the nucleic acid splints are substantially complementary to the polynucleotide molecule, and
at least two of the nucleic acid splints include a sequence which is substantially free of deletions when aligned with the specific sequence.

44. The recording medium according to claim 43, wherein the nucleic acid splints are 8 mer or more complementary to the polynucleotide molecule.

45. The recording medium according to claim 43, wherein a distance between a nucleic acid splint aligned with the specific sequence and a nucleic acid splint adjacent to the nucleic acid splint aligned with the specific sequence is 2 bases or less.

46. The recording medium according to claim 43, wherein the deletions are 2 bases or less.

47. The recording medium according to claim 43, wherein the nucleic acid splint has a sequence that protrudes from a joining point of the polynucleotide fragment molecule.

48. The recording medium according to claim 43, wherein at least one end of the polynucleotide fragment molecule has an association with a carrier.

49. The recording medium according to claim 48, wherein the carrier is polystyrene, CPG (Controlled pore glass), or magnetic beads.
